# NEUE EUROPÄISCHE PATENTSCHRIFT

(11) **EP 2 567 682 B2**
(45) Veröffentlichungstag und Bekanntmachung des Hinweises auf die Entscheidung über den Einspruch: **27.12.2017**
(45) Hinweis auf die Patenterteilung: 13.08.2014
(21) Anmeldenummer: 11007377.2
(22) Anmeldetag: 09.09.2011
(51) Int. Cl.: A61F 13/14, A61M 27/00, A61M 1/00

(54) **Abdominal-Wundauflage mit Applikationshilfe**
Abdominal wound dressing with application aid
Pansement abdominal doté d'une aide à l'application

(43) Veröffentlichungstag der Anmeldung: 13.03.2013
(73) Patentinhaber: Paul Hartmann AG, 89522 Heidenheim (DE)
(72) Erfinder: Croizat, Pierre, Dr., 89542 Herbrechtingen (DE); Eckstein, Axel, Dr., 89522 Heldenheim (DE); Wolf, Cornelia, 89542 Herbrechtingen (DE)

(56) Entgegenhaltungen:
- WO- -96//09795
- WO- -02//074199
- WO- -03//059201
- WO-A1-01/85248
- WO-A1-2010/016791

## Beschreibung

Die vorliegende Erfindung betrifft eine Wundauflage und eine Vorrichtung zur Verwendung bei der Unterdrucktherapie von Wunden im Abdominalbereich. Die Erfindung betrifft weiterhin ein chirurgisches Instrument zur Applikation einer Wundauflage.

Vorrichtungen zur Unterdrucktherapie von Wunden und Wundauflagen als Bestandteil derartiger Vorrichtungen sind im Stand der Technik bekannt.
So beschreibt beispielsweise die WO1993/009727 eine Vorrichtung zur Förderung der Wundheilung durch die Applikation von Unterdruck auf dem die Wunde aufweisenden und die Wunde umgebenden Hautbereich. Die Vorrichtung gemäß WO1993/009727 umfasst eine Vakuumeinrichtung zur Erzeugung des Unterdrucks, eine als Dichtungseinrichtung bezeichnete luftdichte Abdeckung der Wunde, welche mit der Vakuumeinrichtung in einer funktionellen Verbindung steht, sowie eine als Schutzeinrichtung bezeichnete Wundauflage zur Positionierung an der Wunde innerhalb der Dichtungseinrichtung. Bei der Schutzeinrichtung handelt es sich um einen porösen Polymerschaumstoff, beispielsweise um Polyester-Schaum. Ausweislich der Beschreibung von WO1993/009727 kann durch die Anwendung der Unterdrucktherapie die Wundheilung unterschiedlich Typen von Wunden wie beispielsweise Brandwunden, Druckwunden oder Platzwunden beschleunigt werden.

Der Ausdruck "Unterdruck" bezeichnet dabei einen innerhalb des Wundverbandes gegenüber dem Umgebungsluftdruck (atmosphärischer Luftdruck) erniedrigten Luftdruck. Unter "innerhalb des Wundverbandes" wird der durch das luftdichte Abdeckmaterial und dem Körpergewebe im Wundbereich gebildete Zwischenraum (Wundraum) verstanden.

"Unterdruck" wird häufig auch als "negativer Druck" bezeichnet. Die Druckdifferenz zwischen dem Luftdruck innerhalb des Wundverbandes und dem Umgebungsluftdruck wird im Zusammenhang mit der Erfindung in mm Hg (Millimeter-Quecksilbersäule) angegeben, da dies im Bereich der Unterdrucktherapie üblich ist. 1 mm Hg entspricht einem Torr beziehungsweise 133,322 Pa (Pascal). Im Zusammenhang mit der Erfindung wird der Unterdruck, d.h. die Druckdifferenz zwischen dem Luftdruck innerhalb des Wundverbandes und dem Umgebungsluftdruck, als positiver Zahlenwert in mm Hg angegeben.

Besonders großflächige Wunden können im Abdominalbereich entweder infolge von Verletzungen oder infolge von chirurgischen Eingriffen entstehen. Chirurgische Eingriffe im Abdominalbereich werden beispielsweise bei der operativen Behandlung akuter und lebensbedrohlicher Erkrankungen des Bauchraums vorgenommen. Im Rahmen der postoperativen Versorgung derartiger chirurgischer Eingriffe kann auch die Notwendigkeit bestehen, den offenen Abdominalbereich mittels eines temporären Wundverschlusses nur vorübergehend abzudecken.

Freigelegte innere Organe oder das Omentum majus (auch als Bauchnetz oder großes Netz bezeichnet) bilden im Falle einer Abdominalwunde einen Wundgrund, also eine innerhalb eines Wundrandes befindliche Körperoberfläche. Bei der Versorgung einer Abdominalwunde wird eine zur Wunde hin orientierte Schicht der Wundauflage (im Folgenden als Wundkontaktschicht bezeichnet) direkt auf freigelegte innere Organe oder auf das Omentum majus aufgebracht. Die den freigelegten inneren Organen oder dem Omentum majus aufliegende Wundkontaktschicht dient bei der Unterdruck-Behandlung einer Abdominalwunde auch als Organschutzschicht, welche ein unerwünschtes Verkleben von Organen oder dem Omentum majus mit der Bauchdecke verhindern soll. Der Randbereich der Wundauflage wird üblicherweise in den durch Bauchdecke und innere Organe gebildeten Zwischenraum eingeführt.

Während der Behandlung einer Wunde am offenen Abdomen kann es erforderlich sein, eine sehr große Flüssigkeitsmenge, beispielsweise mehr als 5 l innerhalb von 48 Stunden, abzuleiten. Sehr große abzuleitende Flüssigkeitsmengen können z.B. während der operativen Behandlung eines Darmverschlusses (Ileus) oder einer Bauchfellentzündung (Peritonitis) entstehen.

Aus der WO01/85248 ist ein Verband zur temporären Abdeckung von Wunden aus Unfällen oder chirurgischen Eingriffen, insbesondere von Abdominalwunden, bekannt. Der Verband ist zur Verwendung in der Unterdrucktherapie vorgesehen. Die WO01/85248 schlägt vor, den Wundgrund mit einer mit Löchern versehenen Folie abzudecken. Auf die Folie, welche die Wundkontaktschicht darstellt, wird ein poröser Schaum aufgebracht. Auf der von der Wunde abgewandten Seite umfasst der Verband einen für Flüssigkeiten undurchlässigen Abdeckfilm mit Kleberand zum luftdichten Verschließen des Wundbereichs. Weiterhin sind Verbindungsmittel vorgesehen, welche sich durch den Abdeckfilm hindurch bis zum porösen Schaum erstrecken, um den Wundraum mit einer Unterdruckquelle verbinden zu können. Im Betrieb kann Wundexsudat aus dem Wundraum entfernt werden, indem die Flüssigkeit durch die Öffnungen der gelochten Folie zunächst zum porösen Schaum und weiterhin über den Schaum zum Verbindungsmittel gelangt, welches sich im direkten Kontakt mit dem porösen Schaum befindet.

Die WO01/85248 zielt insbesondere auf einen Wundverband, welcher ohne Beschädigung bzw. Traumatisierung der Wunde gewechselt werden kann. Derartige Beschädigungen beim Verbandwechsel können entstehen, wenn während der Therapie Verklebungen zwischen dem Wundverband und dem darunter liegendem Gewebe gebildet werden oder wenn Gewebe in den Verband einwächst, insbesondere beim Einwachsen faserigen Gewebes in den Schaumstoffanteil des Wundverbandes. Die WO01/85248 möchte das unerwünschte Einwachsen von Gewebe in den Verband vermindern, indem der direkte Kontakt porösen Materials mit dem Wundgrund vermindert wird. Die WO01/85248 schlägt dazu vor, dass die Fläche der Öffnungen der mit Löchern versehenen Folie weniger als 10 % der effektiven Fläche der Folie beträgt. Idealerweise sollte die offene Fläche weniger als 1 oder 2 % der Fläche der Folie betragen. Gemäß der WO01/85248 können die Öffnungen in der Folie in Form von Schlitzen vorliegen, was den Kontakt zwischen Schaum und Wundgrund weiter vermindert.

Die WO2010/051068 beschreibt eine Wundauflage für das offene Abdomen, welche eine Vielzahl umhüllter strangartiger Druckverteilungs-Elementen umfasst, die durch ein zentrales Verbindungselement funktionell gekoppelt sind. Auf der von der Wunde abgewandten Seite des zentralen Verbindungselementes ist ein weiteres Druckverteilungs-Element aufgebracht. Bei den Druckverteilungs-Elementen kann es sich beispielsweise um einen offenzelligen Schaumstoff handeln. Die Wundauflage ist zuschneidbar.

Die US2009/0099519 beschreibt einen Abdominalverband für die Unterdrucktherapie, welcher ein umhülltes Polster und ein Heizelement zur Temperaturkontrolle umfasst.

Die WO2010/124844 offenbart eine Wundauflage, welche insbesondere zum Einsatz am offenen Abdomen vorgesehen ist. Die Wundauflage umfasst zwei bahnförmige Elemente, welche einen zwischen ihren inneren Begrenzungsflächen liegenden Drainageraum bilden. Mindestens eines der bahnförmigen Elemente kann Öffnungen aufweisen, wobei insbesondere in den Drainageraum mündende kanalförmige Öffnungen vorgesehen sind, welche eine Kapillarwirkung begünstigen können.

Die Patentanmeldung DE102010052336.4 (zum Zeitpunkt der vorliegenden Anmeldung noch nicht veröffentlicht) des Anmelders der vorliegenden Patentanmeldung beschreibt einen Verband zur Verwendung bei der Unterdrucktherapie von Wunden, insbesondere von Wunden im Abdominalbereich, umfassend eine flexible Folie als Wundkontaktschicht und mindestens ein auf die Folie aufgebrachtes Leitungsmittel aus einem biegsamen elastomeren Material mit einer Dickenerstreckung (H) von höchstens 30 mm. Das Leitungsmittel weist mindestens einen durchgehenden Hohlraum auf. Sowohl Leitungsmittel als auch Folie weisen eine Vielzahl von Öffnungen auf, so dass eine Fluid-Verbindung zwischen dem Leitungsmittel und dem Wundraum herstellbar ist. Die im Leitungsmittel neben den endständigen Öffnungen vorhandenen seitlichen Öffnungen ermöglichen eine Aufnahme von Wundfluid in den durchgehenden Hohlraum. Die Wundauflage kann auf der im Gebrauch von der Wunde abgewandten Seite mindestens eine oder mehrere randständige und zur Mitte der Wundauflage hin offene Taschen mit einer Tiefe von höchstens 6 cm aufweisen, welche das gleichmäßige Aufbringen und Auslegen der Wundauflage auf den Wundgrund erleichtern.

Bei der Applikation einer Abdominalwundauflage auf freigelegte innere Organe oder das Omentum majus muss der Anwender, beispielsweise ein Chirurg, auf eine gleichmäßige Auflage des Verbandes auf den Wundgrund achten. Insbesondere kann es für den Anwender schwierig sein, den umlaufenden Randbereich der Wundauflage zwischen Bauchdecke und innere Organe zu applizieren.

Der vorliegenden Erfindung liegt die Aufgabe zugrunde, eine verbesserte Abdominalwundauflage für die Unterdrucktherapie zur Verfügung zu stellen. Insbesondere ist es Aufgabe der vorliegenden Erfindung, eine in Hinblick auf die Anwendungsfreundlichkeit verbesserte Abdominalwundauflage zu Verfügung zu stellen. Die Wundauflage soll demnach leicht anzulegen, gut an die Gegebenheiten der individuellen Wunde anpassbar und ohne Verklebungen bzw. Verwachsungen mit dem Untergrund zu wechseln sein. Die Wundauflage soll weiterhin eine ausreichende Drainagekapazität aufweisen.

Diese Aufgabe wird erfindungsgemäß gelöst durch eine Wundauflage zur Verwendung bei der Unterdrucktherapie abdominaler Wunden, nach Anspruch 1. Die Wundauflage umfasst eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist. Die erste flexible Folie weist mindestens eine Öffnung auf, um eine Fluid-Kommunikation durch die Folie zu ermöglichen. Des Weiteren umfasst die Wundauflage mindestens eine vorwiegend zur Mitte der Wundauflage hin offene Tasche, welche das gleichmäßige Aufbringen und Auslegen der Wundauflage auf den Wundgrund erleichtert. Die mindestens eine Tasche liegt vorzugsweise randständig vor. Die Tiefe der Tasche beträgt vorzugsweise höchstens 15 cm, insbesondere höchstens 10 cm. Die Tasche wird vorzugsweise durch das Aufbringen tütenartig oder flächig ausgebildeter Materialabschnitte, insbesondere Folienstücke, oder durch die Rückfaltung eines von der Wundauflage umfassten Folienabschnitts gebildet. Erfindungsgemäß unterscheidet sich die vorliegende Erfindung gemäß Anspruch 1 von dem in der Patentanmeldung DE102010052336A1 offenbarten Gegenstand dadurch, dass kein auf die zweite Seite der ersten Folie aufgebrachtes Leitungsmittel aus einem biegsamen elastomeren Material mit einer Dickenerstreckung (H) von höchstens 30 mm, welches einen durchgehenden Hohlraum aufweist vorhanden ist, wobei "Dickenerstreckung (H)" hierbei wie in der Patentanmeldung DE102010052336.4 (zum Zeitpunkt der vorliegenden Anmeldung noch nicht veröffentlicht) dargestellt verstanden werden soll.

Bei der Anwendung in der Unterdrucktherapie von Wunden ist die Wundauflage mit einer Unterdruckquelle Fluid-leitend verbindbar, so dass eine Fluid-Kommunikation zwischen der Unterdruckquelle und dem Wundraum herstellbar ist. Der Abdeckfilm wird üblicherweise auf dem die Wunde umgebenden Hautbereich dichtend befestigt. Unter Fluid werden in diesem Zusammenhang sowohl Flüssigkeiten (beispielsweise Wundfluid, Blut, Darmflüssigkeit oder Spülflüssigkeit), als auch Gase (beispielsweise Luft oder Kohlendioxid) verstanden.

Erfindungsgemäß wird vorgeschlagen, eine Wundauflage mit mindestens einer Tasche zu versehen, wodurch das Applizieren der Wundauflage durch einen Anwender wesentlich erleichtert werden kann. Insbesondere kann der Rand der erfindungsgemäßen Wundauflage einfacher und faltenfrei in den von inneren Organen oder Omentus majus und Bauchdecke gebildeten Zwischenraum appliziert werden. Der Anwender kann dazu ein flaches chirurgisches Instrument, beispielsweise einen Bauch- und Darmspatel, in die Tasche einführen und sodann die durch die Tasche vorübergehend am Spatel gehaltene Wundauflage vorsichtig unter die Bauchdecke einschieben. Nach dem Einschieben des Randbereichs der Wundauflage unter die Bauchdecke wird der Spatel wieder aus der Tasche herausgezogen. Vorzugsweise umfasst die Wundauflage eine Vielzahl von Taschen. Es können dann nacheinander oder gleichzeitig mehrere periphere Anteile der Wundauflage unter die Bauchdecke eingebracht werden, insofern auf der Wundauflage entsprechend angeordnete Taschen vorgesehen sind.

Anstelle eines Instrumentes kann der Anwender zum Applizieren der Wundauflage auch einen oder mehrere Finger in die mindestens eine Tasche einführen.

Die mindestens eine auf der Wundauflage aufgebrachte Tasche kann auch in solchen Fällen die Applikation einer großflächigen Wundauflage erleichtern, in denen es nicht beabsichtigt ist, einen Randbereich der Wundauflage unter die Bauchdecke einzuschieben, so dass sich weitere Verwendungsmöglichkeiten der erfindungsgemäßen Wundauflage ergeben. So hat sich gezeigt, dass auf der Wundauflage aufgebrachte Taschen eine besonders sorgfältige Anpassung der Wundauflage an die hier als Wundgrund bezeichnete Körperoberfläche ermöglichen.

Die erfindungsgemäße Wundauflage umfasst eine erste flexible Folie mit einer ersten und einer zweiten Seite, wobei die erste Seite zum Aufbringen auf den Wundgrund vorgesehen ist. Im Gebrauch wird die flexible Folie mit ihrer ersten Seite in direktem Kontakt auf die hier als Wundgrund bezeichnete Oberfläche, also insbesondere auf die chirurgisch oder verletzungsbedingt freigelegten inneren Organe oder auf das Omentum majus, gelegt. Im Falle einer Abdominalwunde wird der Randbereich der Wundauflage üblicherweise in den durch Wundgrund und Bauchdecke gebildeten Zwischenraum eingebracht. Eine solchermaßen applizierte Wundauflage dient insbesondere bei einem temporalen Wundverschluss als Organschutzschicht und soll darüber hinaus ein Verkleben von Bauchdecke mit dem Wundgrund verhindern. Es ist daher wesentlich, dass die erste flexible Folie aus einem Material besteht, welches während der Dauer der Anwendung nicht mit dem Wundgrund oder der Bauchdecke verklebt oder verwächst. Das Material soll atraumatische Eigenschaften aufweisen.
Geeignete Materialien für die erste flexible Folie umfassen thermoplastische Folien, insbesondere Folien aus Ethylvinylacetat (im Folgenden: EVA), Polyurethan (im Folgenden: PU), Polyethylen (im Folgenden: PE), Polyethylenterephthalat (im Folgenden: PET), PTFE (im Folgenden: Polytetrafluorethylen), Polyvinylchlorid (im Folgenden: PVC), thermoplastische Elastomere (im Folgenden: TPE), Polyorganosiloxan (im Folgenden: Silikon) oder einer Mischung daraus. Unter der Bezeichung TPE sind in diesem Zusammenhang thermoplastische Elastomere auf Olefinbasis (TPO), vernetzte thermoplastische Elastomere auf Olefinbasis (TPV), thermoplastische Elastomere auf Urethanbasis (TPU), thermoplastische Polyesterelastomere bzw. thermoplastische Copolyester (TPC), Styrol-Blockcopolymere (TPS) und thermoplastische Copolyamide (TPA) umfasst. Vorzugsweise handelt es sich bei der thermoplastischen Folie um eine PE-Folie.

Das Flächengewicht der ersten flexiblen Folie beträgt vorzugsweise mindestens 30 g / m² und höchstens 150 g / m², insbesondere mindestens 45 g / m² und höchstens 95 g / m² und besonders bevorzugt mindestens 55 g / m² und höchstens 65 g / m².

Gemäß einer besonders bevorzugten Ausführungsform handelt es sich bei der ersten flexiblen Folie um eine Stützfilm-freie PE-Folie mit einem Flächengewicht von 55 g / m² bis 65 g / m² auf, wobei das Flächengewicht nach der Norm EN ISO 2286 - 2 bestimmt wird, beispielsweise die Produkte "Folie MEDIFOL® 3D, Type 44600" oder "Folie MEDIFOL® 3D T16, Type 44601 T16" der Firma rkw Prolife (Wasserburg, Deutschland).

Die erste flexible Folie weist mindestens eine Öffnungen auf, um eine Unterdruckkommunikation mit dem gesamten Wundraum zu ermöglichen und einen ungehinderten Abfluss von Fluid zu gewährleisten.

Gemäß einer ersten Ausführungsform handelt es sich bei der Öffnung um eine mittig angeordnete Öffnung mit einem Durchmesser von beispielsweise 0,5 cm bis 5,0 cm.

Gemäß einer erfindungsgemäß bevorzugten Ausführungsform weist die erste flexible Folie eine Vielzahl von über die Fläche verteilten Öffnungen auf, welche einen Durchtritt von Wundfluid durch die Folie und eine gleichmäßige Verteilung des Unterdrucks im Wundraumgewährleisten. Bei den Öffnungen kann es sich um Löcher oder um Schlitze handeln. Als Loch wird in diesem Zusammenhang eine Öffnung bezeichnet, welche in der Aufsicht im ungedehnten Zustand eine offene Fläche aufweist. Als Schlitz wird in diesem Zusammenhang eine Öffnung bezeichnet, welche in der Aufsicht im ungedehnten Zustand keine offene Fläche aufweist. Hinsichtlich Form und Größe der Öffnungen kann in geeigneter Weise auf die erwünschte Durchlässigkeit der Folie für Wundfluid abgestellt werden. Denkbar sind runde, ovale, eckige oder beispielsweise sternförmige Löcher. Bei den Schlitzen kann es sich beispielsweise um längliche oder kreuzförmige Schlitze handeln.
Die Öffnungen können gleichmäßig, d.h. in regelmäßig sich wiederholenden Mustern oder zufällig über die Fläche der ersten Folie verteilt vorliegen.

Es ist dabei auch möglich, dass nur ein erster Anteil der ersten flexiblen Folie eine Vielzahl von über die Fläche verteilten Öffnungen aufweist, während ein weiterer Anteil der ersten flexiblen Folie keine Öffnungen aufweist. So kann es beispielsweise vorteilhaft sein, dass ein zentraler Anteil der ersten Folie eine Vielzahl von Öffnungen aufweist, während ein peripherer Anteil der Folie keine Öffnungen aufweist. Eine umgekehrte Anordnung ist gleichfalls möglich.

Falls es sich bei den Öffnungen um Schlitze handelt, sollten diese eine Länge von jeweils mindestens 1 mm und höchstens 30 mm, vorzugsweise mindestens 2 mm und höchstens 20 mm und insbesondere mindestens 5 mm und höchstens 10 mm aufweisen. Dabei hat sich gezeigt, dass eine Folie, welche Schlitze mit einer Länge von mindestens 5 mm und höchstens 10 mm aufweist, wobei zwischen 10 und 90 derartiger Schlitze auf einer FolienFläche von 100 cm² eingebracht werden, besonders vorteilhafte Eigenschaften hinsichtlich Stabilität bei gleichzeitig ausreichender Fluid-Durchlässigkeit aufweist.

Vorzugsweise weist die flexible Folie eine Vielzahl von Öffnungen auf, wobei es sich bei den Öffnungen um Löcher handelt. Der Durchmesser der Löcher kann hinsichtlich der erwünschten Fluid-Durchlässigkeit in geeigneter Weise angepasst werden, beispielsweise bei kreisförmigen Löchern in einem Bereich vom 0,1 mm bis 5 mm. Bereiche zwischen mindestens 0,2 mm und höchstens 0,4 mm sind dabei hinsichtlich einer günstigen Kombination von Fluid-Durchlässigkeit und atraumatischen Eigenschaften der Folie besonders bevorzugt. Löcher mit einem Durchmesser von 0,3 mm weisen hierbei besonders vorteilhafte Eigenschaften auf.

Falls es sich bei den Öffnungen in der ersten Folie um Löcher handelt, soll die Summe der offenen Fläche der Löcher des Weiteren mindestens 0,5 %, vorzugsweise jedoch mindestens 10 % der Flächenerstreckung der Folie betragen, um eine ausreichende Durchlässigkeit der Folie für Wundfluid zu gewährleisten. Dabei soll möglichst eine offene Fläche von 25 % der Folie nicht überschritten werden, da sich dies andernfalls negativ auf die Stabilität der Folie auswirken könnte. Vorzugsweise beträgt die Summe der offenen Fläche der in der Folie vorhandenen Löcher deshalb mindestens 0,5 % und höchstens 25 % der Flächenerstreckung, vorzugsweise mindestens 12 % und höchstens 23 % der Flächenerstreckung.

Fluid-Durchlässigkeit, Stabilität und die Neigung von Gewebe zum Durchwachsen der Folie werden durch die offene Fläche der Folie beeinflusst.

Gemäß einer bevorzugten Ausführungsform der Erfindung beträgt die offene Fläche der in der ersten Folie vorhandenen Löcher mindestens 13 % und höchstens 15 % der Flächenerstreckung der Folie. Eine derartige Folie erweist sich insbesondere in Bezug auf Fluid-Durchlässigkeit, atraumatische Eigenschaften und Stabilität als vorteilhaft. Die Anzahl der in der ersten Folie pro Fläche vorhandenen Öffnungen sollte bei dieser Ausführungsform mindestens 150 pro cm² und höchstens 190 pro cm², insbesondere mindestens 165 pro cm² und höchstens 171 pro cm², betragen.

Gemäß einer weiteren bevorzugten Ausführungsform der Erfindung beträgt die offene Fläche der in der ersten Folie vorhandenen Löcher mindestens 20 % und höchstens 22 % der Flächenerstreckung der Folie. Eine derartige Folie weist eine hohe Fluid-Durchlässigkeit und Flexibilität bzw. Weichheit auf. Die Anzahl der in der ersten Folie pro Fläche vorhandenen Öffnungen sollte bei dieser weiteren Ausführungsform mindestens 260 pro cm² und höchstens 300 pro cm², insbesondere mindestens 275 pro cm² und höchstens 285 pro cm², betragen.

Denkbar ist auch, dass die Folie eine Vielzahl von über die Fläche verteilten Öffnungen aufweist, wobei es sich bei den Öffnungen um eine Kombination von Löchern und Schlitzen handelt. Dies kann beispielsweise derart ausgeführt werden, dass ein bestimmter Flächenbereich der Folie Löcher aufweist und ein anderer Flächenbereich der ersten Folie Schlitze aufweist.
Erfindungsgemäß sind hinsichtlich Form, Größe, Anzahl und Anordnung der Öffnungen in der ersten flexiblen Folie weitere, hier nicht speziell aufgeführte Varianten umfasst.

Gemäß einer besonders bevorzugten Ausführungsform der Erfindung werden die in der ersten Folie vorhandenen Öffnungen durch Perforieren oder Stanzen der Folie derart erzeugt, dass weitestgehend konisch oder zylindrisch geformte Öffnungen entstehen. Die Öffnungen weisen deshalb eine dreidimensionale Struktur auf, so dass die Folie eine glatte Seite und eine der glatten Seite gegenüberliegende aufgeraute Seite aufweist. Das Einbringen von Öffnungen mit einer dreidimensionalen Struktur kann beispielsweise durch eine Stanzwalze erfolgen. Alternativ können Öffnungen mit einer dreidimensionalen Struktur bereits während der Herstellung der Folie erzeugt werden, beispielsweise durch Führung der extrudierten, noch schmelzflüssigen Folie über eine rotierende Unterdruck-Lochwalze.
Besondere Vorteile bei Verwendung einer Folie mit derartigen dreidimensional strukturierten Öffnungen für die erfindungsgemäßen Wundauflage ergeben sich insbesondere dann, wenn die erste, zum Aufbringen auf den Wundgrund vorgesehene Seite der ersten Folie durch die glatte Seite der Folie gebildet wird und die zweite Seite der ersten Folie durch die aufgeraute Seite der Folie gebildet wird. Die zum Aufbringen auf den Wundgrund vorgesehene glatte Seite der Folie weist eine geringe Neigung zum Verkleben mit dem Wundgrund auf. Anderseits bewirken die dreidimensional strukturierten Öffnungen eine Beabstandung des Wundgrundes zu weiteren Lagen des Verbandes, beispielsweise zu einer Schaumstofflage, falls derartige zusätzliche Verbandschichten vorhanden sind. Eine Beabstandung zu weiteren Lagen kann ein unerwünschtes Einwachsen faserigen Gewebes in weitere Verbandlagen vermindern.
Folien, welche konisch oder zylindrisch geformte Öffnungen aufweisen, sind kommerziell erhältlich, beispielsweise die vorgenannten Folien mit den Bezeichnungen "Folie MEDIFOL® 3D, Type 44600" oder "Folie MEDIFOL® 3D T16, Type 44601 T16" (Hersteller rkw ProLife, Wasserburg, Deutschland).

Gemäß einer sehr vorteilhaften Weiterbildung der Erfindung umfasst die Wundauflage weiterhin eine zweite flexible Folie, wobei zumindest ein Anteil der Fläche der ersten Seite der zweiten Folie auf die im Gebrauch von der Wunde abgewandte zweite Seite der ersten Folie aufgebracht wird. Üblicherweise wird die zweite Folie vollflächig mit der ersten Folie in Kontakt gebracht. Es kann jedoch, wie unten näher ausgeführt, vorgesehen sein, dass Materialstücke in den durch die erste Folie und die zweite Folie gebildeten Zwischenraum eingebracht werden, so dass nur Anteile der Fläche der zweiten Folie mit der ersten Folie in direktem Kontakt stehen.

Vorzugsweise ist die zweite Folie aus einem weitestgehend Fluid-undurchlässigen Material gefertigt. Zur Herstellung der zweiten flexiblen Folie können insbesondere die vorstehend bereits im Zusammenhang mit der ersten flexiblen Folie erwähnten Materialien und Fertigprodukte verwendet werden, beispielsweise thermoplastische Folien, insbesondere Folien aus EVA, PU, PE, PET, PTFE, PVC, TPE, Silikon oder einer Mischung daraus.

Ein aus zwei Folienlagen aufgebaute Wundauflage kann hinsichtlich Stabilität und Handhabbarkeit der Wundauflage vorteilhafte Eigenschaften aufweisen. Die Durchlässigkeit der Wundauflage für Wundfluid, sowie die atraumatischen Eigenschaften der Wundauflage können durch eine geeignete Auswahl von erster Folie und zweiter Folie an die therapeutischen Erfordernisse angepasst werden.

Die zweite Folie wird unlösbar auf die zweite Seite ersten Folie aufgebracht, beispielsweise durch Verkleben, Verpressen oder durch Verschweißen. Die Befestigung kann flächig, strichförmig oder punktförmig (Haftpunkte) erfolgen. Ein Verkleben kann beispielsweise durch das Auftragen eines Adhäsivs oder mittels eines Klebebandes erfolgen. Ein Verschweißen kann beispielsweise durch Hitze oder durch Ultraschall erfolgen.

Gemäß einer besonders vorteilhaften Ausführungsform erfolgt die Befestigung der zweiten Folie auf der ersten Folie durch punktförmiges Verkleben oder Verschweißen der Folien, wobei eine Vielzahl von über die Fläche verteilten Haftpunkten vorgesehen ist. Bei einer derartigen Verbindung von erster und zweiter Folie entsteht ein Fluid-leitender Zwischenraum (Labyrinth) zwischen erster und zweiter Folie, welcher den Abfluss von Wundexsudat in vorteilhafter Weise begünstigen kann.

Gemäß einer vorteilhaften Ausführungsform ist die zweite Folie weitestgehend Fluidundurchlässig ausgebildet und weist lediglich eine einzige Öffnung auf. Gleichzeitig sollte die erste flexible Folie eine Vielzahl von über die Fläche verteilten Öffnungen aufweisen, so dass Wundfluid durch die Öffnungen in der ersten Folie in den durch erste Folie und zweite Folie gebildeten Zwischenraum gelangen kann. Eine Weiterleitung des Fluid aus dem Zwischenraum hin zur Unterdruckquelle kann dann über eine vorzugsweise im zentralen Bereich der zweiten Folie angeordnete Öffnung erfolgen.

Gemäß einer weiteren besonders vorteilhaften Ausführungsform weist die erste Folie und die zweite Folie eine Vielzahl von über die Fläche verteilten Öffnungen auf, welche zum Durchleiten von Fluid geeignet sind, wobei die offene Fläche der in der ersten Folie und in der zweiten Folie vorhandenen Löcher jeweils mindestens 0,5 % und höchstens 25 % der Flächenerstreckung betragen sollte. Dabei kann es sich als vorteilhaft erweisen, wenn die in der ersten Folie vorhandenen Öffnungen und die in der zweiten Folie vorhandenen Öffnungen so angeordnet sind, dass die Öffnungen weitestgehend nicht zueinander in Deckung stehen. Unter "weitestgehend nicht zueinander in Deckung stehend" wird in diesem Zusammenhang verstanden, dass zueinander in Deckung stehende Öffnungen von erster und zweiter Folie höchstens zufällig und nur in geringer Anzahl vorhanden sind. Insbesondere sollen dabei mindestens 90% vorzugsweise 95% der Öffnungen in der ersten Folie nicht mit einer Öffnung in der zweiten Folie in Deckung stehen.

Im Zusammenhang mit einer Ausführungsform der erfindungsgemäßen Wundauflage, welche zwei oder mehr Folienlagen umfasst, hat es sich darüber hinaus als besonders vorteilhaft erwiesen, einen auch bei Vorliegen eines Unterdrucks weitgehend stabilen Hohlraum zwischen erster und zweiter Folie vorzusehen. Ein derartiger weitgehend stabiler Hohlraum kann geschaffen werden, indem erste und zweite Folie voneinander beabstandet aufeinander gebracht werden. Dies kann beispielsweise durch das Einbringen geeigneter Materialstücke zwischen die Folien erfolgen. Bei dem Materialstück kann es sich im einfachsten Fall um bloße Abstandshalter handeln, beispielsweise um Noppen aus Kunststoff. Denkbar sind des Weiteren Materialstücke, welche Fluid-leitende Eigenschaften aufweisen, beispielsweise Materialstücke aus Schaumstoff. Eine Beabstandung der Folienschichten unter Ausbildung eines Hohlraumes kann auch durch die Verwendung eines Folienmaterials erreicht werden, welches dreidimensional geformte Öffnungen aufweist. Dazu müssen die Folien so aufeinander gebracht werden, dass die dreidimensionalen Strukturen einander zugewandt vorliegen.
In diesem Zusammenhang wird auf den Inhalt der bereits zitierten Patentanmeldung WO2010/124844 Bezug genommen, welche eine Wundabdeckung aus zwei bahnförmigen Elementen beschreibt. Die bahnförmigen Elemente weisen trichterförmige Perforationen auf und bilden einen zwischen ihren inneren Begrenzungsflächen liegenden Drainageraum, wobei ein Kapillareffekt auftritt. Dabei können zwei Bahnen mit einander zugewandten Trichteröffnungen übereinander gelegt und punktförmig miteinander verbunden werden. Entsprechend wird gemäß einer weiteren vorteilhaften Ausführungsform der Erfindung vorgeschlagen, auf die in der WO/124844 offenbarte Wundabdeckung mindestens eine vorwiegend zur Mitte der Wundauflage hin offene Tasche auf die im Gebrauch von der Wunde abgewandten Seite der Wundauflage aufzubringen.

Die vorgenannte Ausführungsform der Erfindung betrifft somit eine Wundauflage zur Verwendung bei der Unterdrucktherapie von Wunden, insbesondere zur Behandlung abdominaler Wunden, umfassend
i) eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist, und wobei die erste Folie weiterhin eine Vielzahl von über die Fläche verteilten und weitestgehend konisch oder zylindrisch geformten Öffnungen mit einer dreidimensionalen Form aufweist,
ii) eine zweite flexible Folie mit einer ersten und einer zweiten Seite, wobei die erste Seite der zweiten Folie auf die zweite Seite der ersten Folie aufgebracht ist und wobei die zweite Folie weiterhin eine Vielzahl von über die Fläche verteilten und weitestgehend konisch oder zylindrisch geformten Öffnungen aufweist, und
iii) mindestens eine, vorzugsweise eine Vielzahl von vorwiegend zur Mitte der Wundauflage hin offener Taschen, welche auf der im Gebrauch von der Wunde abgewandten Seite der Wundauflage vorhanden sind und welche das gleichmäßige Aufbringen und Auslegen der Wundauflage auf den Wundgrund erleichtern. Die Tiefe der mindestens einen Tasche beträgt vorzugsweise höchstens 15 cm, insbesondere höchstens 10 cm.
Dabei sollen erste und die zweite Folie so aufeinander gebracht werden, dass die dreidimensionalen Strukturen einander zugewandt vorliegen und ein zwischen ihren inneren Begrenzungsflächen liegenden Drainageraum gebildet wird.
Weiterhin ist die Wundauflage ist dadurch gekennzeichnet, dass kein auf die zweite Seite der ersten Folie aufgebrachtes Leitungsmittel aus einem biegsamen elastomeren Material mit einer Dickenerstreckung (H) von höchstens 30 mm vorhanden ist, welches einen durchgehenden Hohlraum aufweist, vorhanden ist.
Die mindestens eine Tasche kann dabei insbesondere einen tütenartig ausgebildeten Materialabschnitt oder einen flächigen Materialabschnitt umfassen.

Es ist in Fortbildung dieses Gedankens auch möglich, ein Fluid-leitendes Material, beispielsweise einen Schaum, zwischen erster und zweiter Folie anzuordnen. Die bereits erwähnte Patentanmeldung WO2010/051068, auf deren Inhalt hiermit Bezug genommen wird, beschreibt eine solchermaßen gestaltete Abdominal-Wundauflage. Die Wundauflage der WO2010/051068 umfasst eine Vielzahl umhüllter strangartiger Druckverteilungs-Elemente, welche beispielsweise aus einem offenzelligen Schaum bestehen können. Die Druckverteilungs-Elemente sind auf eine erste Folie, welche zum Kontakt mit dem Körpergewebe vorgesehen ist, aufgebracht. Die Druckverteilungs-Elemente werden seitlich und wundabseits von einer weiteren zweiten Folienlage umhüllt. Die zweite Folie steht in den zwischen den Strängen lokalisierten Abschnitten der Wundauflage in unlösbarer Verbindung mit der ersten Folie. Erste und zweite Folie weisen Öffnungen auf.
Es wird in diesem Zusammenhang weiterhin auf die ebenso bereits erwähnte Patentanmeldung WO01/85248 Bezug genommen. Die WO01/85248 schlägt vor, den Wundgrund mit einer mit Löchern versehenen Folie abzudecken, auf die ein poröser Schaum aufgebracht wird. Der poröse Schaum ist wundabseits von einer weiteren Folienlage mit Öffnungen überfangen, so dass der Schaum in einem durch erste und zweite Folie gebildeten Zwischenraum gehalten wird. Die Folienlagen sind seitlich unlösbar miteinander verbunden.
Im Rahmen der vorliegenden Erfindung können die in den Patentanmeldungen WO20101051068 und WO01/85248 beschriebenen Wundauflagen mit mindestens einer vorwiegend zur Mitte der Wundauflage hin offenen Tasche versehen werden, um das gleichmäßige Aufbringen und Auslegen der Wundauflage auf dem Wundgrund zu erleichtern.

Die vorgenannte weitere Ausführungsform der Erfindung betrifft somit eine Wundauflage zur Verwendung bei der Unterdrucktherapie von Wunden, insbesondere zur Behandlung abdominaler Wunden, umfassend
i) eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist, und wobei die erste Folie weiterhin eine Vielzahl von über die Fläche verteilten Öffnungen aufweist,
ii) eine zweite flexible Folie mit einer ersten und einer zweiten Seite, wobei die erste Seite der zweiten Folie auf die zweite Seite der ersten Folie aufgebracht ist und wobei die zweite Folie weiterhin eine Vielzahl von über die Fläche verteilten Öffnungen aufweist,
iii) mindestens ein zwischen erste Folie und zweite Folie eingebrachtes Materialstück, so dass ein zwischen den inneren Begrenzungsflächen der Folien liegender Drainageraum gebildet wird, und
iii) mindestens eine, vorzugsweise eine Vielzahl von vorwiegend zur Mitte der Wundauflage hin offener Taschen, welche auf der im Gebrauch von der Wunde abgewandten Seite der Wundauflage vorhanden sind und welche das gleichmäßige Aufbringen und Auslegen der Wundauflage auf den Wundgrund erleichtern. Die Tiefe der mindestens einen Tasche beträgt vorzugsweise höchstens 15 cm, insbesondere höchstens 10 cm. Weiterhin ist die Wundauflage ist dadurch gekennzeichnet, dass kein auf die zweite Seite der ersten Folie aufgebrachtes Leitungsmittel aus einem biegsamen elastomeren Material mit einer Dickenerstreckung (H) von höchstens 30 mm vorhanden ist, welches einen durchgehenden Hohlraum aufweist, vorhanden ist.

Die mindestens eine Tasche kann dabei insbesondere einen tütenartig ausgebildeten Materialabschnitt oder einen flächigen Materialabschnittes umfassen. Bei dem mindestens einen zwischen erste Folie und zweite Folie eingebrachten Materialstück handelt es sich vorzugsweise um einen offenporigen Schaumstoff, insbesondere um einen offenporigen Polyurethanschaumstoff. Polyester-Polyurethane sind hierbei besonders bevorzugt, wobei insbesondere auf den in der Patentanmeldung DE 102010034819.8 (zum Zeitpunkt der vorliegenden Anmeldung noch nicht veröffentlicht) beschriebenen Schaumstoff verwiesen wird.

Auf eine zweite flexible Folie können gegebenenfalls weitere wundabseitig angeordnete Folienlagen aufgebracht werden. Nach einer weiteren vorteilhaften Ausführungsform der Erfindung umfasst die Wundauflage somit eine oder mehrere weitere flexible Folienlagen, wobei die eine oder die die mehreren Folienlagen zum Aufbringen auf die im Gebrauch von der Wunde abgewandte Seite der Wundauflage vorgesehen sind.

Zur Herstellung der einen oder mehreren weiteren flexiblen Folienlagen können insbesondere die vorstehend bereits im Zusammenhang mit der ersten flexiblen Folie erwähnten Materialien und Fertigprodukte verwendet werden, beispielsweise thermoplastische Folien, insbesondere Folien aus EVA, PU, PE, PET, PTFE, PVC, TPE, Silikon oder einer Mischung daraus. Hinsichtlich der Befestigung der einen oder mehreren weiteren flexiblen Folie auf der zweiten flexiblen Folie wird auf die oben stehenden Vorschläge zur Befestigung der zweiten Folie auf der ersten Folie verwiesen.

Durch die Verwendung weiterer Folienlagen kann die Stabilität und die Durchlässigkeit der Wundauflage für Wundfluid weiter an die therapeutischen Erfordernisse angepasst werden.

Zur Aufrechterhaltung der Drainagekapazität der Wundauflage über einen Zeitraum von mehreren Stunden wird im Zusammenhang mit einer weiteren vorteilhaften Ausgestaltung der Erfindung vorgeschlagen, die vor der Wundauflage umfassten Folienmaterialien mit einer anti-koagulierend wirkenden Substanz zu versehen. Hiermit kann beispielsweise eine Verstopfung der Öffnungen oder des - falls vorhanden - durch erste und zweite Folie gebildeten Zwischenraumes reduziert werden. Gemäß einer solchen besonders vorteilhaften Ausführungsform weist die erste flexible Folie und/oder die zweite flexible Folie (falls vorhanden) und/oder die eine oder mehreren weiteren flexiblen Folienlagen (falls vorhanden) eine Beschichtung oder Imprägnierung mit einer antikoagulierend wirkenden Substanz (beispielsweise Heparin, oder eine andere, üblicherweise zur Beschichtung von medizinischen Oberflächen oder Röhrchen verwendete Substanz mit antikoagulierender Wirkung) auf. Es ist gleichfalls denkbar die zur Bildung einer Tasche vorgesehenen Materialabschnitte mit einer derartigen Beschichtung oder Imprägnierung zu versehen.

Erfindungsgemäß umfasst die Wundauflage mindestens eine vorwiegend zur Mitte der Wundauflage hin offene Tasche, welche das gleichmäßige Aufbringen und Auslegen der Wundauflage auf den Wundgrund erleichtert. Die mindestens eine Tasche ist dabei vorzugsweise randständig auf der Wundauflage angeordnet. Unter randständig wird hier verstanden, dass die Tasche überwiegend im peripheren Anteil der Wundauflage angebracht wird. Der Abstand des äußeren Randes der Tasche zum Rand der Wundauflage könnte bei einer in diesem Sinne als randständigen bezeichneter Anordnung auf einer kreisförmigen Wundauflage mit einem Durchmesser von 45 cm beispielsweise 0 cm bis 10 cm betragen. Die Tiefe der mindestens einen Tasche beträgt vorzugsweise höchstens 15 cm, insbesondere höchstens 10 cm. Die mindestens eine Tasche wird auf die im Gebrauch von der Wunde abgewandte Seite (wundabseitig) derjenigen Folienlage aufgebracht, welche im Gebrauch von der Wunde abgewandt vorliegt: Insofern die Wundauflage lediglich eine erste flexible Folie umfasst, wird die Tasche auf die zweite Seite der ersten Folie aufgebracht. Insofern die Wundauflage lediglich aus zwei Folienlagen besteht, d.h. aus erster flexibler Folie und zweiter flexibler Folie, wird die Tasche im Allgemeinen auf die zweite Seite der zweiten Folie aufgebracht. Eine Ausnahme kann sich hierbei ergeben, wenn die zweite Folie nur einen Anteil der Fläche der ersten Folie überfängt. In diesem Falle wäre es auch möglich, die Tasche auf demjenigen Anteil der ersten Folie aufzubringen, welcher nicht von der zweiten Folie bedeckt ist. Insofern die Wundauflage eine oder mehrere weitere flexible Folienlagen umfasst, wird die Tasche im Allgemeinen auf die im Gebrauch von der Wunde abgewandte Seite derjenigen Folienlage aufgebracht, welche im Gebrauch von der Wunde abgewandt vorliegt. Dies bedeutet, dass die Tasche stets wundabseitig auf der Wundauflage vorgesehen ist.
Im Rahmen der Erfindung geeignete Taschen sind in einer Vielzahl von Formen vorstellbar. Wesentlich ist dabei, dass eine vorwiegend zur Mitte der Wundauflage hin orientierte Öffnung vorhanden ist, in welche das chirurgische Instrument, insbesondere ein Bauch- und Darmspatel, eingeschoben bzw. eingehakt werden kann. Weiterhin ist wesentlich, dass eine der Öffnung gegenüberliegende Seite der Tasche dem eingeschoben bzw. eingehakten chirurgischen Instrument einen Widerhalt zu geben vermag. Am einfachsten ist ein derartiger Widerhalt durch eine der Öffnung der Tasche gegenüberliegende Naht zu realisieren. Die Öffnung der Tasche sollte vorwiegend zur Mitte der Wundauflage hin orientiert sein, damit ein vom Zentrum der Wundauflage her nach außen bewegtes chirurgisches Instrument in die Öffnung eingeschoben werden kann und dort einen Widerhalt zu finden vermag.

Eine vorwiegend zur Mitte der Wundauflage hin orientiert Öffnung der Tasche kann gleichermaßen eine manuelle Applikation der Wundauflage erleichtern, falls der Anwender anstelle eines chirurgischen Instrumentes einen Finger in die mindestens eine Tasche einführen möchte.

Die Tasche sollte hinsichtlich ihrer Form und Größe auf das chirurgische Instrument oder gegebenenfalls auf einen menschlichen Finger abgestimmt sein. Die Tasche kann beispielsweise im Wesentlichen die Form eines Rechtecks, eines Trapez, eines Halbrunds, eines Dreiecks, eines Kreisrings oder eines Kreisringsektors aufweisen, wobei weitere hier nicht näher beschriebene Formen, die sich für den Fachmann aus der Haltefunktion der Tasche ergeben, umfasst sind.

Die Tiefe a der Tasche, also der Abstand der Öffnung der Tasche bis zum gegenüberliegenden Rand der Tasche, sollte vorzugsweise höchstens 15 cm betragen, insbesondere höchstens 10 cm. Die Tiefe a sollte mindestens 0,3 cm, vorzugsweise jedoch mindestens 1 cm betragen, damit das in die Tasche eingeführte chirurgische Instrument oder ein gegebenenfalls in die Tasche eingeführter Finger beim Applizieren der Wundauflage nicht aus der Tasche herausrutschen kann. Eine erfindungsgemäß geeignete Tasche weist deshalb vorzugsweise eine Tiefe von 1 cm bis 10 cm auf.
Hinsichtlich der Breite b der Tasche ist es gleichfalls erforderlich, diese auf das bei der Applikation der Wundauflage zu Anwendung kommende chirurgische Instrument abzustimmen, weshalb die Breite b der Tasche mindestens der Breite des Instruments aufweisen muss, da dieses andernfalls nicht in die Tasche eingeführt werden kann. Es ist jedoch vorteilhaft, die Tasche wesentlich breiter als das Instrument auszulegen, da bei einer solchermaßen gestalteten Tasche das Einführen des Instrumentes in die Tasche einfacher bewerkstelligt werden kann. Der Anwender muss in diesem Falle nicht darauf achten, eine vergleichsweise enge Öffnung der Tasche zu finden, was sich im Falle einer stark blutenden Wunde als schwierig erweisen kann. Die Breite b der Tasche beträgt im Allgemeinen mindestens 1 cm, vorzugsweise mindestens 2 cm.

Erfindungsgemäß umfasst die Wundauflage mindestens eine auf der im Gebrauch von der Wunde abgewandten Seite der Wundauflage (wundabseits) vorhandene Tasche. Es erweist sich jedoch als besonders vorteilhaft für die Applikation der Wundauflage, wenn die Wundauflage eine Vielzahl von Taschen umfasst, welche an verschiedenen Stellen der Wundauflage angebracht sind. Hierbei wird vorgeschlagen dass die Wundauflage mindestens 3 Taschen umfasst, vorzugsweise mindestens 4, insbesondere mindestens 6 Taschen. Die Taschen sollten in einem weitestgehend gleichmäßigen Abstand zueinander auf der Wundauflage angebracht werden. Die Taschen können insbesondere auf einem oder mehreren konzentrischen, den Mittelpunkt der Wundauflage umlaufenden Kreisen angeordnet vorliegen, wobei die Taschen vorzugsweise auf jedem Kreis mit einem zueinander gleichmäßigen Abstand verteilt vorliegen. Vorzugsweise liegen die Taschen auf mindestens 30 % des Umfangs der vorgenannten konzentrisch Kreis vor, insbesondere auf mindestens 50 % des Umfangs.

Die erfindungsgemäße Wundauflage kann anwenderseitig, also seitens des Arztes oder des medizinischen Personals, uneingeschränkt an die Form und Größe der Wunde angepasst werden, beispielsweise durch einfaches Zuschneiden mit einer sterilen Schere. Insofern es dagegen erwünscht ist, das Produkt bereits herstellerseitig an vordefinierte Wundformen und Wundgrößen angepasst bereit zu stellen, so ergeben sich durch den Aufbau der erfindungsgemäßen Wundauflage in dieser Hinsicht gleichfalls keine Einschränkungen. Hinsicht einer Zuschneidbarkeit der Wundauflage durch den Anwender ist es dabei besonders vorteilhaft, wenn die Taschen auf der Wundauflage in mehreren, konzentrischen, den Mittelpunkt der Wundauflage umlaufenden Kreisen angeordnet vorliegen. Wenn bei einer derartigen Anordnung die weiter außen angebrachten Taschen bei Zuschneiden abgeschnitten oder zerstört werden, so können bei der Applikation der Wundauflage die weiter innen vorhandenen Taschen weiter verwendet werden.

Eine bevorzugte Ausführungsform der erfindungsgemäßen Wundauflage umfasst mindestens eine Tasche, wobei die Tasche durch das Aufbringen von flächigen Materialabschnitten, insbesondere Folienstücken, auf die im Gebrauch von der Wunde abgewandte Seite der Wundauflage gebildet wird. Die Tasche kann insbesondere durch Verkleben, thermisches Verschweißen, Verpressen oder Ultraschallschweißen auf der Wundauflage befestigt werden. Die Befestigung an der Wundauflage erfolgt am Rand des Materialabschnittes, so dass eine Außennaht hergestellt wird. Der vorwiegend zur Mitte der Wundauflage hin orientierte Rand des Materialabschnittes wird dabei nicht mit der Wundauflage verbunden, so dass eine Öffnung zum Einschieben eines chirurgischen Instrumentes oder eines Fingers vorhanden ist. Eine derartige Tasche ähnelt in ihrem Aufbau einer Hemdtasche, also einer nach drei Seiten mit einer Naht verschlossenen Tasche, welche eine Öffnung aufweist, in die ein Gegenstand eingeführt werden kann. Die im Gebrauch zur Wunde hin zeigende innere Oberfläche der Tasche wird dabei durch den wundabseitig angeordneten Folienanteil der Wundauflage gebildet, während die im Gebrauch von der Wunde weg zeigende innere Oberfläche der Tasche durch den aufgebrachten Materialabschnitt gebildet wird. Eine derartige Tasche kann auf einfache und kostengünstige Weise hergestellt werden.

Gemäß einer alternativen und gleichfalls sehr vorteilhaften Ausgestaltung der Erfindung weist die Wundauflage wundabseits eine oder mehrere Taschen auf, welche aus einem Materialabschnitt in der Form eines Kreisrings gefertigt sind. Eine solchermaßen ausgeführte Tasche eignet sich insbesondere zum Anbringen auf eine kreisförmige Abdominalwundauflage. Der die Form eines Kreisrings aufweisende Materialabschnitt wird an seinem äußeren Umfang an der Wundauflage befestigt, so dass eine kreisförmige Naht gebildet wird. Durch jeden Materialabschnitt wird jeweils eine einzige, die Mitte der Wundauflage umlaufende Tasche mit einer zur Mitte der Wundauflage hin zeigenden Öffnung gebildet. Der äußere Umfang des Kreisrings darf dabei höchstens die Größe der Wundauflage aufweisen. Der innere Umfang des Kreisrings sollte vorzugsweise so gewählt werden, dass eine Tasche mit einer Tiefe von höchstens 15 cm, insbesondere höchstens 10 cm gebildet wird. Es können mehrere konzentrisch angeordnete derartige Taschen auf die Wundauflage aufgebracht werden. Dies erleichtert die Applikation einer durch Zuschneiden an die Wundgröße angepassten Wundauflage: Nach einer Zerstörung einer weiter außen angebrachten Tasche durch das Zuschneiden kann bei der Applikation der Wundauflage eine weiter innen vorhandene Tasche verwendet werden.
Die vorgenannte Ausführungsform ist dabei nicht auf exakt kreisförmig ausgebildete Wundauflagen beschränkt, sondern kann bei geeigneter Anpassung des Materialabschnittes ebenso bei oval gestalteten Abdominalwundauflagen ausgeführt werden.

Im Zusammenhang mit einer durch flächige Materialabschnitte gebildeten Tasche ist die vorliegende Erfindung auf ein Verfahren zur Herstellung einer Wundauflage gerichtet, umfassend die Schritte
a) Bereitstellen einer ersten flexiblen Folie mit einer ersten und einer zweiten Seite, wobei die erste Seite zum Aufbringen auf den Wundgrund, insbesondere auf freigelegte innere Organe oder auf das Omentum majus, vorgesehen ist und wobei die Folie mindestens eine Öffnung aufweist,
b) Bereitstellen mindestens eines flächigen Materialabschnittes, insbesondere mindestens eines Folienstückes, und
c) Aufbringen des mindestens einen flächigen Materialabschnittes auf die zweite Seite der ersten Folie, so dass mindestens eine vorwiegend zur Mitte der Wundauflage hin offene Tasche gebildet wird.

Erfindungsgemäß ist dabei kein auf die zweite Seite der ersten Folie aufgebrachtes Leitungsmittel aus einem biegsamen elastomeren Material mit einer Dickenerstreckung (H) von höchstens 30 mm, welches einen durchgehenden Hohlraum aufweist, vorhanden.

Gemäß einer vorteilhaften Weiterbildung der Erfindung kann die Wundauflage, wie bereits an früherer Stelle im Text dargelegt, eine zweite flexible Folie umfassen. Solchenfalls umfasst das Verfahren zur Herstellung einer Wundauflage
a) Bereitstellen einer ersten flexiblen Folie mit einer ersten und einer zweiten Seite, wobei die erste Seite zum Aufbringen auf den Wundgrund, insbesondere auf freigelegte innere Organe oder auf das Omentum majus, vorgesehen ist und wobei die Folie mindestens eine Öffnung aufweist,
b) Bereitstellen einer zweiten flexiblen Folie mit einer ersten und einer zweiten Seite, wobei die erste Seite zum Aufbringen auf die im Gebrauch von der Wunde abgewandte zweite Seite der ersten Folie vorgesehen ist,
c) Bereitstellen mindestens eines flächigen Materialabschnittes, insbesondere mindestens eines Folienstückes,
d) Aufbringen der ersten Seite der zweiten flexiblen Folie auf die zweite Seite der ersten flexiblen Folie, und
d) Aufbringen des mindestens einen flächigen Materialabschnittes auf die zweite Seite der zweiten Folie, so dass mindestens eine vorwiegend zur Mitte der Wundauflage hin offene Tasche gebildet wird.
Erfindungsgemäß ist dabei kein auf die zweite Seite der ersten Folie aufgebrachtes Leitungsmittel aus einem biegsamen elastomeren Material mit einer Dickenerstreckung (H) von höchstens 30 mm, welches einen durchgehenden Hohlraum aufweist, vorhanden.

Gemäß einer weiteren vorteilhaften Weiterbildung der Erfindung kann die Wundauflage eine oder mehrere weitere flexible Folien, welche zum Aufbringen auf die im Gebrauch von der Wunde abgewandte Seite der Wundauflage vorgesehen sind, umfassen. Das vorstehend für eine zweilagige Wundauflage beschriebene Verfahren wird solchenfalls derart angepasst, dass die mindestens eine Tasche auf diejenige Folienlage aufgebracht wird, welche im Gebrauch von der Wunde abgewandt vorliegt.

Eine weitere bevorzugte Ausführungsform der erfindungsgemäßen Wundauflage umfasst mindestens eine sackartig bzw. tütenartig ausgebildete Tasche, welche zum Aufbringen auf die im Gebrauch von der Wunde abgewandte Seite der Wundauflage vorgesehen ist. Die Tasche sollte dabei so auf die Wundauflage aufgebracht werden, dass deren Öffnung vorwiegend zur Mitte der Wundauflage hin orientiert vorliegt. Die Tasche ist vorzugsweise aus einem Folienmaterial gefertigt und sollte weiterhin vorzugsweise eine Tiefe a von höchstens 15 cm, insbesondere höchstens 10 cm aufweisen. Die Tasche kann insbesondere durch Verkleben, thermisches Verschweißen, Verpressen oder Ultraschallschweißen auf der Wundauflage befestigt werden. Die im Gebrauch zur Wunde hin zeigende innere Oberfläche der Tasche wird bei dieser Ausführungsform durch einen ersten Materialabschnitt der Tüte gebildet, während die im Gebrauch von der Wunde weg zeigende innere Oberfläche der Tasche durch einen zweiten Materialabschnitt der Tüte gebildet wird.

Die vorliegende Erfindung ist deshalb gleichfalls auf ein Verfahren zur Herstellung einer Wundauflage wie vorstehend beschrieben gerichtet, umfassend die Schritte
a) Bereitstellen einer ersten flexiblen Folie mit einer ersten und einer zweiten Seite, wobei die erste Seite zum Aufbringen auf den Wundgrund, insbesondere auf freigelegte innere Organe oder auf das Omentum majus, vorgesehen ist und wobei die Folie mindestens eine Öffnung aufweist,
b) optional Bereitstellen einer zweiten flexiblen Folie mit einer ersten und einer zweiten Seite, wobei die erste Seite zum Aufbringen auf die im Gebrauch von der Wunde abgewandte zweite Seite der ersten Folie vorgesehen ist,
c) optional Bereitstellen einer oder mehrerer weiterer flexibler Folien, welche zum Aufbringen auf die im Gebrauch von der Wunde abgewandte Seite der Wundauflage vorgesehen sind,
d) Bereitstellen mindestens einer sackartig bzw. tütenartig ausgebildeten Tasche, insbesondere einer sackartig bzw. tütenartig ausgebildeten Tasche aus einem Folienmaterial,
e) gegebenfalls Verbinden der ersten flexiblen Folie mit der zweiten flexiblen Folie und gegebenenfalls Verbinden der zweiten flexiblen Folie mit den einen oder mehreren weiteren flexibler Folien, und
d) Aufbringen der mindestens einen sackartig bzw. tütenartig ausgebildeten Tasche auf die im Gebrauch von der Wunde abgewandte Seite der Wundauflage derart, dass die Öffnung der Tasche vorwiegend zur Mitte der Wundauflage hin zeigt.

Erfindungsgemäß ist dabei kein auf die zweite Seite der ersten Folie aufgebrachtes Leitungsmittel aus einem biegsamen elastomeren Material mit einer Dickenerstreckung (H) von höchstens 30 mm, welches einen durchgehenden Hohlraum aufweist, vorhanden. Die Tiefe der mindestens einen Tasche beträgt vorzugsweise höchstens 15 cm, insbesondere höchstens 10 cm.

Gemäß einer weiteren bevorzugten Ausführungsform umfasst die erfindungsgemäße Wundauflage mindestens eine Tasche, wobei die Tasche durch Rückfaltung eines Folienanteils der Wundauflage gebildet wird. Die Tasche wird dabei insbesondere durch Rückfaltung der ersten Folie und/oder der zweiten flexiblen Folie und/oder der einen oder mehreren weiteren flexiblen Folien gebildet. Im Folgenden werden beispielhaft drei mögliche Ausführungen i) bis iii) für eine derartige, durch Rückfaltung gebildete Tasche aufgezeigt. Die Erfindung ist nicht auf die hier vorgeschlagenen Beispiele beschränkt. Für den Fachmann ergibt sich eine Vielzahl weiterer Möglichkeiten, den hier vorgestellten Gedanken einer durch Rückfaltung gebildeten Tasche umzusetzen.
i) Herstellung einer Tasche durch Rückfaltung, wobei die Wundauflage kreisförmig ausgebildet ist und nur eine einzige flexible Folie umfasst: In den Rand der ersten flexiblen Folie werden mindestens zwei radial geführte Einschnitte mit einer Tiefe von ca. 3 cm eingebracht. Der Abstand der Einschnitte am Folienrand beträgt ca. 5 cm. Der von den Einschnitten begrenzte Abschnitt der ersten flexiblen Folie wird auf seine zweite Seite (wundabseits) rückgefaltet, wobei der Abschnitt seitlich unter Bildung einer Naht fixiert wird. Eine solchermaßen hergestellte Tasche weist eine Tiefe von ca. 3 cm und eine Breite b von ca. 5 cm auf, wobei eine zur Mitte der Wundauflage hin orientierte Öffnung vorhanden ist.
ii) Herstellung einer Tasche durch Rückfaltung, wobei die Wundauflage kreisförmig ausgebildet ist und neben einer ersten flexiblen Folie eine zweite flexible Folie umfasst: Die erste Folie wird nicht eingeschnitten bzw. rückgefaltet. Die Rückfaltung der zweiten flexiblen Folie erfolgt, wie unter i) für die erste flexible Folie beschrieben. Die Tasche wird in diesem Falle durch die zweite flexible Folie gebildet. Die Tasche ist randständig auf der Wundauflage angeordnet, wobei der Abstand der Tasche zum Rand der Wundauflage 3 cm beträgt.
iii) Herstellung einer Tasche durch Rückfaltung, wobei die Wundauflage kreisförmig ausgebildet ist und neben einer ersten flexible Folie und einer zweiten flexible Folie eine weitere flexible Folie umfasst: In die weitere flexible Folie werden drei Einschnitte so eingebracht, dass eine rechteckig geformte Klappe entsteht, wobei der die äußere Seite des Rechtecks einen Abstand zum Rand der Wundauflage von 5 cm aufweist. Durch Rückfaltung der Klappe nach innen kann eine Tasche gebildet werden, wobei der rückgefaltete Abschnitt seitlich unter Bildung einer Naht fixiert wird.

Die vorliegende Erfindung ist deshalb weiterhin auf ein Verfahren zur Herstellung einer Wundauflage wie vorstehend beschrieben gerichtet, umfassend die Schritte
a) Bereitstellen einer ersten flexiblen Folie mit einer ersten und einer zweiten Seite, wobei die erste Seite zum Aufbringen auf den Wundgrund, insbesondere auf freigelegte innere Organe oder auf das Omentum majus, vorgesehen ist und wobei die Folie mindestens eine Öffnung aufweist,
b) optional Bereitstellen einer zweiten flexiblen Folie mit einer ersten und einer zweiten Seite, wobei die erste Seite zum Aufbringen auf die im Gebrauch von der Wunde abgewandte zweite Seite der ersten Folie vorgesehen ist,
c) optional Bereitstellen einer oder mehrerer weiterer flexibler Folien, welche zum Aufbringen auf die im Gebrauch von der Wunde abgewandte Seite der Wundauflage vorgesehen sind, und
d) Rückfaltung eines Folienanteils der Wundauflage, insbesondere Rückfaltung der ersten Folie und/oder der zweiten flexiblen Folie und/oder der einen oder mehreren weiteren flexiblen Folien, so dass mindestens eine vorwiegend zur Mitte der Wundauflage hin offene Tasche gebildet wird, welche auf der im Gebrauch von der Wunde abgewandten Seite der Wundauflage vorhanden ist und welche das gleichmäßige Aufbringen und Auslegen der Wundauflage auf den Wundgrund erleichtert.

Erfindungsgemäß ist dabei kein auf die zweite Seite der ersten Folie aufgebrachtes Leitungsmittel aus einem biegsamen elastomeren Material mit einer Dickenerstreckung (H) von höchstens 30 mm, welches einen durchgehenden Hohlraum aufweist, vorhanden. Die Tiefe der mindestens einen Tasche beträgt vorzugsweise höchstens 15 cm, insbesondere höchstens 10 cm.

Zur Gewährleistung einer ausreichenden Drainagekapazität der Wundauflage kann optional vorgesehen sein, dass die Tasche aus einem Material, insbesondere aus einem Folienmaterial gefertigt wird, welches eine Vielzahl von über die Fläche verteilten Öffnungen aufweist. Vorzugsweise sollten die in dem zur Bildung einer Tasche vorgesehenen Materialabschnitt vorhandenen Öffnungen mindestens 0,1 % und höchstens 25 % der Flächenerstreckung, vorzugsweise mindestens 10 % und höchstens 22 % der Flächenerstreckung des Materialabschnitts aufweisen.

Zur Applikation einer erfindungsgemäßen Wundauflage kann der Anwender ein flaches chirurgisches Instrument, beispielsweise einen Bauch- und Darmspatel, in die mindestens eine Tasche einführen und sodann die durch die Tasche vorübergehend am Spatel gehaltene Wundauflage vorsichtig unter die Bauchdecke einschieben. Alternativ wäre es auch möglich, zum Applizieren der Wundauflage anstelle eines Instrumentes einen oder mehrere Finger in die mindestens eine Tasche einzuführen.

In einer vorteilhaften Weiterführung der Erfindung wird vorgeschlagen zur Applikation der Wundauflage ein speziell angepasstes chirurgisches Instrument einzusetzen. Es wurde gefunden, dass ein Instrument aus einem flexiblen Kunststoffmaterial auf besonders schonende Weise zur Applikation der Wundauflage verwendet werden kann und insofern gegenüber einem aus Metall gefertigten Instrument Vorteile aufweist. Unter einem flexiblen Kunststoffmaterial wird in diesem Zusammenhang verstanden, dass die Shore A Härte des Materials höchstens 80, insbesondere höchstens 70 beträgt (bestimmt nach DIN 53505 vom August 2000, und zwar bei 23°C an einem wie in der Norm beschrieben plattenförmigen ebenen und glatten Probekörper einer Dicke von 6 mm). Das Instrument weist ein Spatel-artig ausgebildetes Endstück auf, welches in eine Tasche der erfindungsgemäßen Wundauflage einführbar ist. Aus Sicherheitsgründen sollte das Instrument zudem ein Mittel zum Erzeugen eines Röntgenkontrasts umfassen, so dass ein im Wundraum zurückgelassenes Instrument auf einer Röntgenaufnahme detektiert werden kann. Bei dem Mittel zum Erzeugen eines Röntgenkontrasts kann es sich beispielsweise um einen Röntgenkontraststreifen, einen Röntgenkontrastfaden oder einen Röntgenkontrastchip handeln, welcher auf das Instrument aufgebracht wird. Es wäre auch möglich eine Substanz, welche einen Röntgenkontrast erzeugen kann, in das zur Herstellung des Spatels verwendete Material einzuarbeiten.

Die Erfindung betrifft somit weiterhin ein speziell angepasstes chirurgisches Instrument zur Applikation einer Wundauflage, wobei die Wundauflage
i) eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist, und wobei die erste Folie weiterhin mindestens eine Öffnung aufweist,
ii) mindestens eine vorwiegend zur Mitte der Wundauflage hin offene Tasche, welche auf der im Gebrauch von der Wunde abgewandten Seite der Wundauflage vorhanden ist und welche das gleichmäßige Aufbringen und Auslegen der Wundauflage auf den Wundgrund erleichtert, umfasst. Das chirurgische Instrument weist dabei ein Spatel-artig ausgebildetes, in die Tasche der Wundauflage einführbares Endstück auf und umfasst weiterhin ein flexibles Kunststoffmaterial sowie ein Mittel zum Erzeugen eines Röntgenkontrasts.

Die Erfindung betrifft gleichfalls die Verwendung eines speziell angepassten chirurgischen Instrumentes zur Applikation einer Wundauflage, wobei das chirurgische Instrument ein Spatel-artig ausgebildetes, in die Tasche der Wundauflage einführbares Endstück aufweist, und wobei das Instrument weiterhin ein flexibles Kunststoffmaterial und ein Mittel zum Erzeugen eines Röntgenkontrasts umfasst. Die Wundauflage umfasst dabei
i) eine erste flexible Folie mit einer ersten und einer zweiten Seite, wobei die erste Seite zum Aufbringen auf den Wundgrund, insbesondere auf freigelegte innere Organe oder auf das Omentum majus, vorgesehen ist, und wobei die erste Folie weiterhin mindestens eine Öffnung aufweist,
ii) mindestens eine vorwiegend zur Mitte der Wundauflage hin offene Tasche, welche auf der im Gebrauch von der Wunde abgewandten Seite der Wundauflage vorhanden ist und welche das gleichmäßige Aufbringen und Auslegen der Wundauflage auf den Wundgrund erleichtert.

In der Praxis erweist sich weiterhin als sehr vorteilhaft, wenn die Wundauflage zusätzlich eine oder mehrere flüssigkeitsdurchlässige Schichten zum Aufbringen auf die im Gebrauch von der Wunde abgewandte Seite (wundabseits) der Wundauflage umfasst. Insofern eine derartige flüssigkeitsdurchlässige Schicht vorgesehen ist, wird diese also wundabseits auf die mit mindestens einer Tasche versehene Folienlage aufgebracht. Bei der Folienlage handelt es sich, wie oben dargestellt, um die erste Folie, um die zweite Folie oder um eine weitere Folie.
Die flüssigkeitsdurchlässige Schicht umfasst vorzugsweise einen porösen Schaumstoff, insbesondere einen porösen Polymerschaumstoff. Besonders geeignet ist dabei ein offenzelliger Polymerschaumstoff. Im Rahmen dieser Anmeldung bedeutet der Begriff offenzellig, dass im Schaumstoff (c) mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind.
Geeignete Materialien für einen porösen Schaumstoff umfassen beispielsweise Polyurethan, Polyurethan-Polyharnstoff-Copolymere, Polyvinylalkohol (PVA) oder Silikon.

Alternativ oder zusätzlich kann die flüssigkeitsdurchlässige Schicht textile Materialien wie Woven oder Non-woven umfassen, beispielsweise einen Vliesstoff aus synthetischen Polymeren wie Polyamid, Polyester oder Polypropylen.

Im Zusammenhang mit der vorliegenden Erfindung können die in der deutschen Patentanmeldung DE102010034819.8 (zum Zeitpunkt der vorliegenden Anmeldung noch nicht veröffentlicht) beschriebenen porösen Schaumstoffe in besonders vorteilhafter Weise zur Herstellung der einen oder mehreren flüssigkeitsdurchlässigen Schichten eingesetzt werden. Auf den Inhalt der deutschen Patentanmeldung DE102010034819.8 wird hiermit Bezug genommen. Die in der DE102010034819.8 beschriebenen Schaumstoffe setzen beim zur Anpassung an die Wundform gegebenenfalls nötigen Zuschneiden keine oder nur im geringen Maße Schaumstoffpartikel frei. Freigesetzte Schaumstoffpartikel, welche in die Wunde gelangen, können die Wunde reizen und die Wundheilung beeinträchtigen.
In besonders vorteilhafter Weise kann der vom Anmelder Paul Hartmann AG (Heidenheim, Deutschland) vertriebene offenzellige Polyurethan-Schaumstoff VivanoMed als flüssigkeitsdurchlässige Schicht eingesetzt werden.

Die mindestens eine weitere flüssigkeitsdurchlässige Schicht kann die Weichheit und Verträglichkeit der Wundauflage verbessern und einen zusätzlichen Beitrag zur Ableitung von Wundexsudat leisten.

Weiterhin ist es zur Förderung des primären Wundverschlusses insbesondere sehr vorteilhaft einen porösen Polymerschaumstoff derart anzubringen, dass der Schaumstoff in direktem Kontakt mit den Wundrändern steht.

Die mindestens eine weitere flüssigkeitsdurchlässige Schicht weist eine Dicke von 2 mm bis 50 mm, vorzugsweise von 3 mm bis 30 mm auf.

Die weitere flüssigkeitsdurchlässige Schicht, welche vorzugsweise einen porösen Polymerschaumstoff umfasst, kann dabei die gesamte Fläche der Folie überfangen. Vorzugsweise liegt die flüssigkeitsdurchlässige Schicht nur auf einem mittigen Anteil der Folie vor. Insbesondere bei der Verwendung der Wundauflage als temporärer Wundverschluss hat es sich als günstig erwiesen, wenn ein poröser Polymerschaumstoff derart aufgebracht wird, dass die Ränder des Schaumstoffs in direktem Kontakt mit den Wundrändern stehen.

Im Zusammenhang mit der vorliegenden Erfindung kann auch mehr als eine flüssigkeitsdurchlässige Schicht auf der im Gebrauch von der Wunde abgewandten zweiten Seite der Wundauflage vorgesehen sein, insbesondere mehr als eine Schicht eines porösen Polymerschaumstoffs. Die mehreren Schichten können dabei unterschiedliche Abmessungen aufweisen und in unterschiedlichen Dicken vorliegen. Für die Unterdruckbehandlung einer typischen Abdominalwunde hat es sich als vorteilhaft erwiesen, wenn zwei Schichten eines offenzelligen Polyurethanschaum mit einer Schichtdicke von jeweils 16 mm appliziert werden.

Die erfindungsgemäße Wundauflage kann weiterhin ein luftundurchlässiges Abdeckmaterial zum luftdichten Verschließen der Wunde und der Wundumgebung umfassen. Unter "luftdichten Verschließen" soll hier nicht verstanden werden, dass keinerlei Gasaustausch zwischen dem Wundraum und seiner Umgebung stattfindet. Vielmehr ist mit "luftdichten Verschließen" in diesem Zusammenhang gemeint, dass unter Berücksichtigung der eingesetzten Unterdruckpumpe der für die Unterdrucktherapie von Wunden nötige Unterdruck aufrechterhalten werden kann. Es können deshalb auch Abdeckmaterialien eingesetzt werden, welche eine geringfügige Gas-Permeabilität aufweisen, so lange der für die Unterdrucktherapie notwendige Unterdruck aufrechterhalten werden kann.

Das Abdeckmaterial wird in der Wundumgebung oder am Wundrand so befestigt, dass ein luftdichter Wundverschluss gewährleistet ist. Dabei kann es zweckmäßig sein, wenn das Abdeckmaterial vollflächig selbstklebend ausgestattet ist oder einen selbstklebenden Rand aufweist. Alternativ können Befestigung und Abdichtung beispielsweise mit einer Klebefolie, mit einem flüssigen Kleber oder mit einer Abdichtmasse erfolgen.

In einer bevorzugten Ausführungsform der Erfindung umfasst das Abdeckmaterial zum luftdichten Verschließen der Wunde ein wasserunlösliches Polymer, oder eine Metallfolie.
In einer besonders bevorzugten Ausführungsform der Erfindung handelt es sich bei dem wasserunlöslichen Polymer um Polyurethan, Polyester, Polypropylen, Polyethylen, Polyamid oder Polyvinylchlorid, Polyorganosiloxan (Silikon), oder um eine Mischung daraus.
Als Abdeckmaterial können auch Fertigprodukte eingesetzt werden, welche die vorstehend genannten Eigenschaften aufweisen. Als besonders geeignetes Abdeckmaterial für die erfindungsgemäße Vorrichtung hat sich Polyurethanfilm der Marke Hydrofilm® (Paul Hartmann AG, Deutschland) oder Visulin ® (Paul Hartmann AG, Deutschland) erwiesen.

Im Zusammenhang mit der erfindungsgemäßen Wundauflage kann des Weiteren ein Unterdruck-Anschlussstück zur funktionellen Verbindung des Wundraums mit einer außerhalb der Wundauflage befindlichen Unterdruckquelle verwendet werden, wobei das Unterdruck-Anschlussstück derart gestaltet ist, dass ein Unterdruck im Wundraum herstellbar ist und Flüssigkeiten aus dem Wundraum absaugbar sind. Das Unterdruck-Anschlussstück wird bei der Verwendung der Wundauflage in der Unterdrucktherapie von Wunden vorzugsweise auf die von der Wunde abgewandte Seite des luftundurchlässigen Abdeckmaterials angebracht, wobei das Abdeckmaterial geeignete Öffnungen aufweist. Unterdruck-Anschlussstücke sind dem Fachmann auch unter der Bezeichnung "Port" bekannt. Das Unterdruck-Anschlussstück umfasst üblicherweise eine Verbindungsleitung und einen Unterdruckadapter, um mit den weiteren Komponenten des Unterdrucksystems verbindbar zu sein.

Gemäß einer weiteren bevorzugten Ausführungsform werden Abdeckmaterial und das Mittel zur funktionellen Verbindung des Wundraums mit einer außerhalb des Abdeckmaterials befindlichen Unterdruckquelle bereits gebrauchsfertig miteinander verbunden zur Verfügung gestellt. Es ist ganz besonders bevorzugt, dass diese Ausführungsform eine Folie aus einem oder mehreren wasserunlöslichen Polymeren enthält, welche einen selbstklebenden Rand aufweist, da diese Anordnung das Anlegen des Verbandes wesentlich erleichtert.

Für die in der vorliegenden Erfindung beschriebene Wundauflage sind die in den Patentanmeldungen WO2011091947, WO2011091952 und WO2011076340, sowie die in der deutschen Patentanmeldung DE102011108726.9 (zum Zeitpunkt der vorliegenden Anmeldung noch nicht veröffentlicht) offenbarten Unterdruck-Anschlussstücke besonders geeignet.

Gemäß einer alternativen Ausführungsform kann die funktionelle Verbindung des Wundraums mit einer außerhalb des Abdeckmaterials befindlichen Unterdruckquelle mit mindestens einer Verbindungsleitung hergestellt werden. Die mindestens eine Verbindungsleitung kann durch das Abdeckmaterial hindurchgeführt werden oder unter dem Rand des Abdeckmaterials durchgeführt werden. In beiden Fällen ist die Durchtrittsstelle luftdicht abzudichten, so dass der gewünschte Unterdruck im Verband aufrechterhalten werden kann. Als Abdichtmittel eignet sich beispielsweise eine Klebefolie, eine Klebemasse, oder ein Klebestreifen.

Bei der Verbindungsleitung kann es sich beispielsweise um einen Schlauch, um ein Rohr oder um einen anderen Körper mit einem Hohlraum handeln. Ein geeigneter Schlauch ist beispielsweise ein Silikon-Drainageschlauch.

Die erfindungsgemäße Wundauflage kann weiterhin ein Mittel umfassen, so dass der in der Vorrichtung tatsächlich vorhandene Unterdruck überprüfbar und gegebenenfalls einstellbar ist. Das Mittel kann sich im Wundraum oder an einer anderen geeigneten Stelle befinden. Alternativ ist es auch möglich einen Drucksensor in der Unterdruckleitung zwischen Wundverband und der Unterdruckquelle anzubringen.

Es ist vorgesehen, dass die vorgenannten Komponenten den die Wundbehandlung durchführenden Ärzten und Fachkräften als gebrauchsfertiges Set ("Kit") zur Verfügung gestellt werden. Die Erfindung bezieht sich daher auch auf ein gebrauchsfertiges Set zur Unterdruck-Wundbehandlung umfassend
a) eine Wundauflage nach einem oder mehreren der Ansprüche 1 bis 16,
b) optional mindestens eine flüssigkeitsdurchlässige Schicht zum Aufbringen auf die im Gebrauch von der Wunde abgewandte zweite Seite der Wundauflage, wobei die mindestens eine flüssigkeitsdurchlässige Schicht bevorzugt eine oder mehrere flächenförmige Polster aus einem porösen Polymerschaumstoff, insbesondere aus PU, PVA oder Silikon umfasst,
c) ein luftundurchlässiges Abdeckmaterial zum luftdichten Verschließen der Wunde und der Wundumgebung, wobei das Abdeckmaterial vorzugsweise einen Kleberand aufweist,
d) optional ein Unterdruck-Anschlussmittel zur funktionellen Verbindung des Wundraums mit einer außerhalb des Abdeckmaterials befindlichen Unterdruckquelle, so dass ein Unterdruck im Wundraum herstellbar ist und Flüssigkeiten aus dem Wundraum absaugbar sind, wobei das Unterdruck-Anschlussmittel vorzugsweise zum Anbringen auf die im Gebrauch von der Wunde abgewandte Außenseite des Abdeckmaterials vorgesehen ist,
und wobei die Komponenten a) bis d) steril verpackt vorliegen können.

Für das gebrauchsfertige Set besonders geeignete Unterdruck-Anschlussstücke sind in den vorgenannten Patentanmeldungen Anmeldenummer WO2011091947, WO2011091952, WO2011076340 und DE102011108726.9 (zum Zeitpunkt der vorliegenden Anmeldung noch nicht veröffentlicht) beschrieben.

Weiterhin kann das Set optionale Bestandteile wie beispielsweise eine oder mehrere zusätzliche flächenförmige Elemente einer flüssigkeitsdurchlässigen Schicht, Klebemittel zum Fixieren des Verbands, Abdichtmittel zur Herstellung einer luftundurchlässigen Abdichtung des Verbandes, Drucksensoren, Verbindungselemente für Drucksensoren, Schläuche, Verbindungsstücke für Schläuche, Desinfektionsmittel, Hautpflegemittel, pharmazeutische Zubereitungen oder eine Gebrauchsanweisung enthalten. Dem Set kann weiter optional ein oder mehrere in der Chirurgie übliche Darm- und Bauchspatel oder alternativ ein oder mehrere speziell zur Applikation einer erfindungsgemäßen Wundauflage angepasste chirurgische Instrumente beigefügt werden. Ein speziell zur Applikation einer erfindungsgemäßen Wundauflage angepasstes chirurgisches Instrument weist ein Spatel-artig ausgebildetes, in die Tasche der Wundauflage einführbares Endstück auf. Das Instrument ist aus einem flexiblen Kunststoffmaterial hergestellt und umfasst einen Röntgenkontrastfaden.

Das Set ist zur Verwendung mit einer Unterdruckquelle vorgesehen. Eine besonders geeignete Unterdruckquelle stellt eine Unterdruckeinheit, insbesondere eine tragbare Unterdruckeinheit dar. Eine tragbare Unterdruckeinheit kann Komponenten wie beispielsweise eine Pumpe, ein oder mehrere Flüssigkeitsbehälter, eine Steuereinheit, eine Stromversorgung, elektrische Anschlussmitteln und Schläuche enthalten. Alternativ kann es sich bei der Unterdruckquelle beispielsweise um eine Vorrichtung zur funktionellen Verbindung des Unterdruckverbandes mit einer vorhandenen stationären Unterdruckquelle handeln.

Für das gebrauchsfertige Set besonders geeignete tragbare Unterdruck-Einheiten sind in den Patentanmeldungen WO2011018133 und WO2011018132 beschrieben. Ein für das Set besonders geeignete Unterdruck-Einheit ist unter der Bezeichnung VivanoTec (Hersteller Paul Hartmann AG, Heidenheim, Deutschland) kommerziell erhältlich.

Vorzugsweise werden alle Komponenten, bei denen dies aus medizinischer Sicht notwendig ist, steril abgepackt zur Verfügung gestellt. Der Vorteil des gebrauchsfertigen Sets besteht darin, dass der Unterdruckverband schnell, standardisiert und unkompliziert angelegt werden kann. Ein weiterer Vorteil besteht darin, dass alle im Wundbereich eingesetzten Bestandteile des Sets bereits sterilisiert zur Verfügung gestellt werden können.

### Figurenlegende

- 1.: Erste flexible Folie
- 4.: Zweite flexible Folie
- 6.: Öffnung in erster flexibler Folie
- 7.: Haft- bzw. Befestigungspunkt zwischen erster und zweiter Folie
- 10.: Wundauflage zur Verwendung bei der Unterdrucktherapie von Wunden, insbesondere zur Behandlung abdominaler Wunden
- 11.: Flüssigkeitsdurchlässige Schicht
- 12.: Zwischenraum zwischen Wundgrund (beispielweise Omentum majus oder freigelegte innere Organe) und Bauchdecke
- 13.: Wundgrund (beispielsweise Omentum majus oder freigelegte innere Organe)
- 14.: Bauchdecke
- 15.: Wundrand
- 16.: Luftundurchlässiger Abdeckfilm
- 17.: Öffnung in Abdeckfilm
- 18.: Unterdruck-Anschlussmittel (Port)
- 19.: Unterdruckleitung
- 20.: Kanister für Wundexsudat
- 21.: Unterdruckquelle
- 25.: Zur Mitte der Wundauflage hin offene Tasche
- 26.: Naht bzw. Verklebungsbereich von Tasche mit erster oder zweiter flexibler Folie
- 27.: Öffnung der Tasche
- 28.: Klebeschicht zur Befestigung einer Tasche
- 40.: Vorrichtung zur Verwendung bei der Unterdrucktherapie von Wunden, insbesondere zur Behandlung abdominaler Wunden

### Figuren

Nachstehend wird die erfindungsgemäße Wundauflage bzw. Vorrichtung zur Unterdrucktherapie von Wunden anhand schematischer Zeichnungen näher erläutert (nicht maßstabsgetreu). Die Erfindung soll jedoch nicht auf die in den Zeichnungen oder in der Beschreibung der Zeichnung dargestellten Ausgestaltungen reduziert verstanden werden. Vielmehr umfasst die erfindungsgemäße Vorrichtung auch Kombinationen der Einzelmerkmale der alternativen Formen.
Fig. 1 zeigt eine Ausführungsform der erfindungsgemäßen Wundauflage zur Verwendung bei der Unterdrucktherapie in der Aufsicht auf die wundabgewandte Seite der Wundauflage. Der Ausschnitt zeigt eine vergrößerte Ansicht einer Tasche mit Bemaßung.
Fig. 2 zeigt eine weitere Ausführungsform der erfindungsgemäßen Wundauflage in der Aufsicht auf die wundabgewandte Seite der Wundauflage.
Fig. 3 a -d zeigen weitere beispielhaft Ausführungsformen der erfindungsgemäßen Wundauflage in der Aufsicht auf die wundabgewandte Seite der Wundauflage. Die gezeigten Ausführungsformen unterscheiden sich hinsichtlich der Anordnung der Taschen.
Fig. 4 zeigt einen Ausschnitt der in Fig. 1 dargestellten einlagigen Wundauflage im Querschnitt, entsprechend der Linie A-A von Fig. 1.
Fig. 5 zeigt eine weitere Ausführungsform der Wundauflage mit zwei Folienlagen im Querschnitt. Der Schnitt wurde durch die Mitte der Wundauflage geführt.
Fig. 6 zeigt eine weitere Ausführungsform der Wundauflage mit zwei Folienlagen im Querschnitt. Die Ausführungsform unterscheidet sich von der in Fig. 5 gezeigten Ausführungsform anhand der Taschen. Der Schnitt wurde durch die Mitte der Wundauflage geführt.
Fig. 7 zeigt eine weitere Ausführungsform der Wundauflage mit einer Folienlage und einer tütenförmig ausgebildeten Tasche. Der Schnitt wurde durch die Mitte der Wundauflage geführt.
Fig. 8 zeigt eine an eine abdominale Wunde angelegte Vorrichtung zur Verwendung bei der Unterdrucktherapie von Wunden im Querschnitt. Die von der Vorrichtung umfasste Wundauflage weist den in Fig. 1 und Fig. 4 gezeigten Aufbau auf. Der Schnitt wurde durch die Mitte der Wundauflage geführt, entsprechend der Linie A-A von Fig. 1.

### Ausführliche Beschreibung der Erfindung

Die Figuren zeigen verschiedene Ausführungsformen und Ansichten der erfindungsgemäßen, insgesamt mit dem Bezugszeichen 10 bezeichneten Wundauflage.

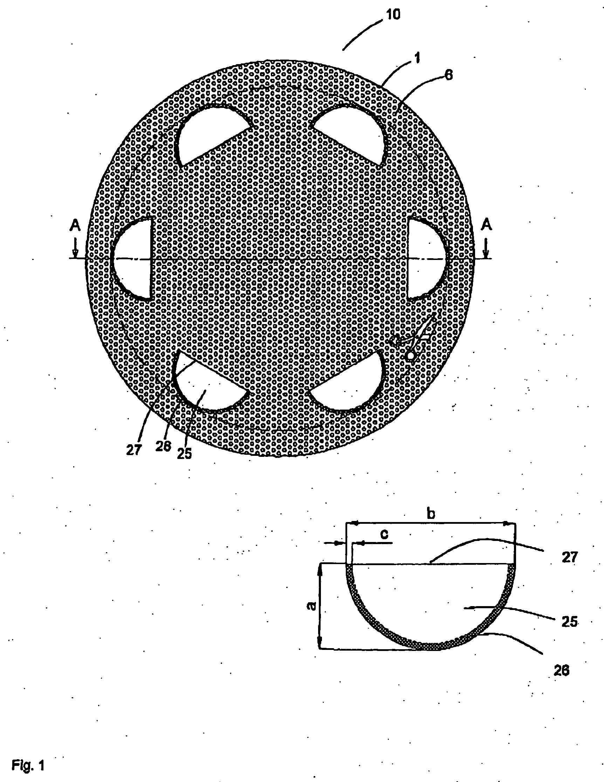

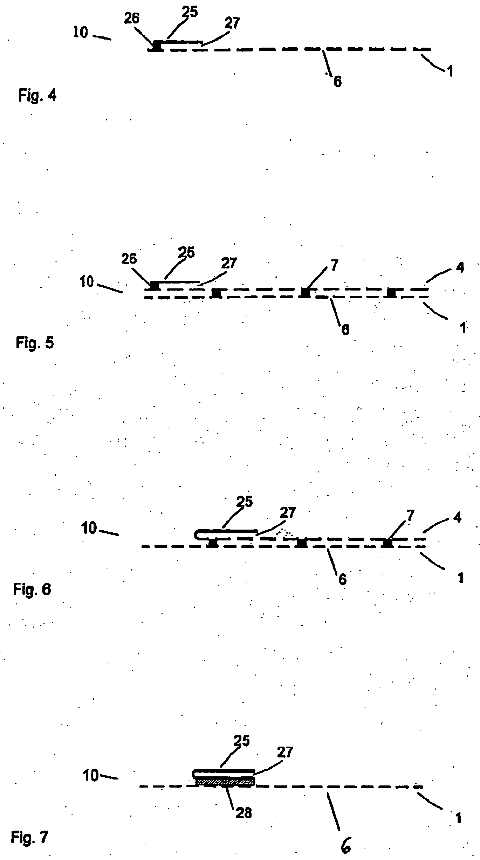

Fig. 1 zeigt eine Ausführungsform, welche hier beispielhaft lediglich eine erste flexible Folie 1 umfasst. Die erste flexible Folie 1 weist eine erste und einer zweite Seite auf, wobei die erste Seite zum Aufbringen auf den Wundgrund (siehe Fig. 7, Bezugszeichen 13), insbesondere auf freigelegte innere Organe oder auf das Omentum majus, vorgesehen ist und somit als Wundkontaktschicht und gegebenenfalls als Organschutzschicht dienen kann. Die flexible Folie 1 weist weiterhin eine Vielzahl von über die Fläche verteilten Öffnungen 6 auf, wobei die offene Fläche der in der Folie vorhandenen Öffnungen 14 % beträgt. Bei den Öffnungen handelt es sich um kreisförmige Löcher mit einem Durchmesser von ca. 0,3 mm. Gemäß einer weiteren vorteilhaften Ausführungsform, welche im Vergleich zur vorgenannten Folie 1 weicher und durchlässiger ist, beträgt die offene Fläche der in der Folie 1 vorhandenen Öffnungen 6 (kreisförmige Löcher mit einem Durchmesser von 0,3 mm) 21 %. Generell sollte die Summe der offenen Fläche der in der Folie 1 vorhandenen Öffnungen 6 mindestens 0,1 % und höchstens 25 % der Flächenerstreckung, vorzugsweise mindestens 10 % und höchstens 22 % der Flächenerstreckung betragen.

Auf die Folie 1 sind an einem kreisförmigen inneren Umfang sechs Taschen 25 auf der zweiten Seite der ersten Folie 1 angeordnet. Die Taschen sind auf ungefähr 58 % des Kreisumfangs vorhanden und auf dem Umfang mit zueinander gleichmäßigem Abstand verteilt. Die Taschen erleichtern das gleichmäßige Aufbringen und Auslegen der Wundauflage 10 auf den Wundgrund 13. Die Taschen weisen bei der in Fig. 1 dargestellten Ausführung eine insgesamt halbkreisförmige Form auf. Die Taschen sind an einem randständigen Anteil der Wundauflage 10 angebracht. Der Abstand einer Tasche 25 zum Rand der Wundauflage 10 beträgt 4 cm. Die gerade Seite des Halbkreises bildet eine zur Mitte der Wundauflage 10 hin zeigende Öffnung 27, in welche bei der Applikation der Wundauflage auf den Wundgrund 13 ein chirurgisches Instrument eingebracht oder ein Finger eingeschoben werden kann. Mittels der Naht 26 ist die Tasche 25 mit der ersten Folie unlösbar verbunden. Dies kann beispielsweise durch Verkleben oder Verschweißen bewerkstelligt werden. Die Tasche 25 ist bei dem in Fig. 1 gezeigten Beispiels aus einem flächigen Materialabschnitt gefertigt, beispielsweise aus einer Kunststofffolie. Der die Tasche 25 bildende Materialabschnitt kann über die Fläche des Materialabschnitts verteilte Öffnungen aufweisen (nicht dargestellt). Damit kann eine über die Fläche der Wundauflage 10 gleich bleibende Durchlässigkeit für Fluid gewährleistet werden. Die Breite b der Tasche (siehe vergrößerter Ausschnitt in Fig. 1) beträgt im Allgemeinen mindestens 1 cm und höchstens 20 cm, vorzugsweise mindestens 2 cm und höchstens 10 cm. Im Falle der in Fig. 1 gezeigten halbkreisförmigen Taschen 25 beträgt die Breite b beispielsweise 6 cm und die Tiefe a dann 3cm. Die Breite c der Naht kann im Bereich von 0,1 mm bis 10 mm liegen.

Durch das Scherensymbol in Fig. 1 soll angedeutet werden, dass die Wundauflage 10 durch Zuschneiden auf die für die Behandlung der Wunde benötigte Größe angepasst werden kann.

Falls die Wundauflage 10 wie im vorliegenden Beispiel lediglich eine einzige flexible Folie 1 umfasst, werden die Taschen 25 auf die zweite Seite der ersten Folie 1 aufgebracht. Falls die Wundauflage 10 zweilagig ausgestaltet ist, werden die Taschen 25 im Allgemeinen auf die zweite Seite der zweiten Folie aufgebracht. Im Falle einer Wundauflage 10, welche darüber hinaus weitere Schichten umfasst, sind die Taschen 25 auf der im Gebrauch von der Wunde abgewandten Seite derjenigen Folienlage vorhanden, welche im Gebrauch von der Wunde abgewandt vorliegt.

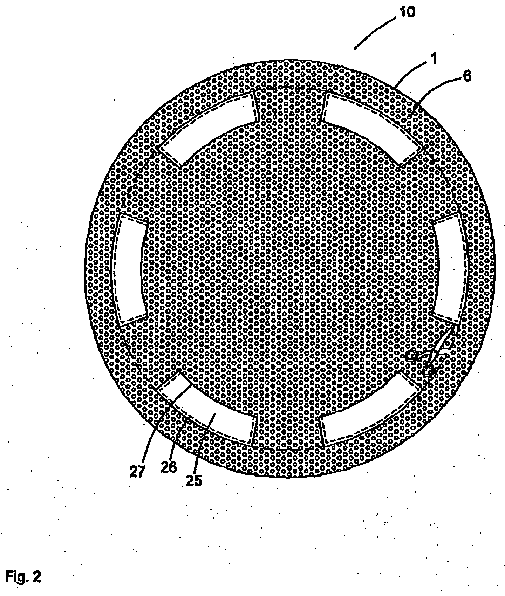

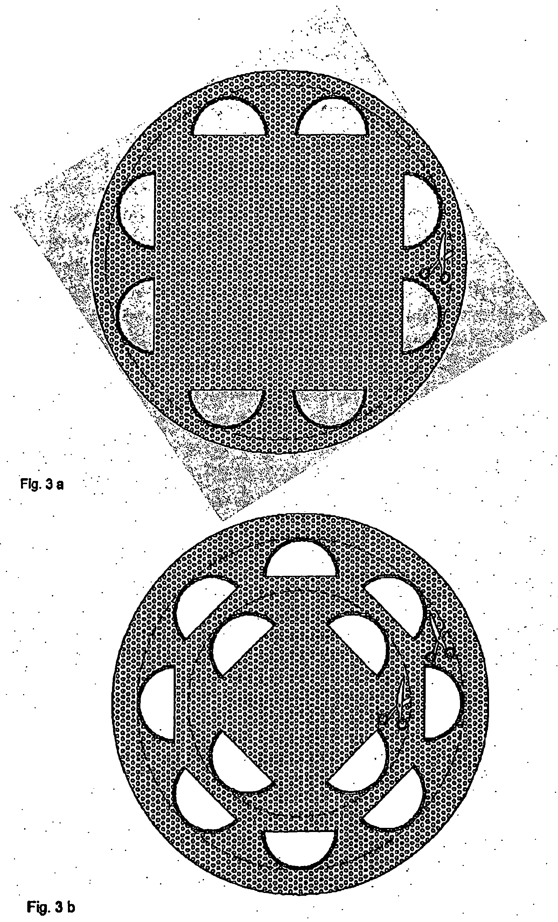

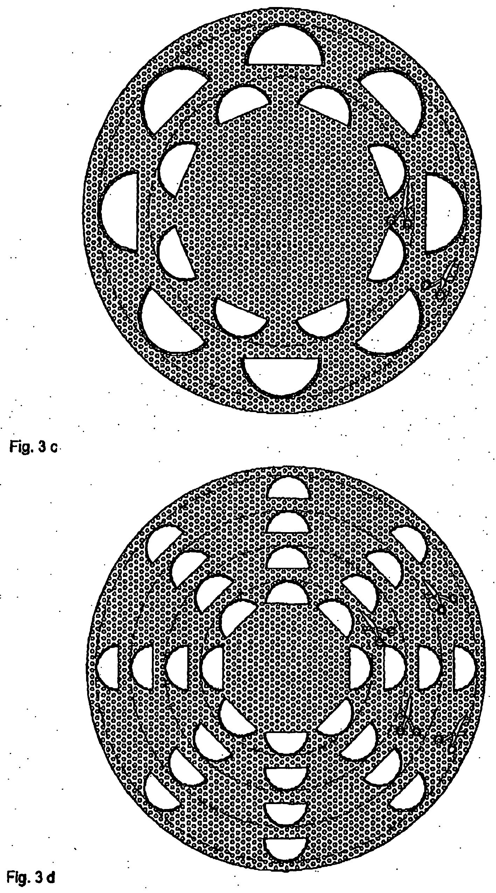

Mit Fig. 2 wird eine ansonsten zu Fig. 1 identische Wundauflage 25 gezeigt, welche sich jedoch hinsichtlich der Form der Taschen 25 unterscheidet. Die in Fig. 2 schematisch dargestellten Taschen 25 weisen die Form eines Kreisringsektors auf. Eine nicht mit der Folie 1 verbundene Seite der Tasche zeigt jeweils zur Mitte der Wundauflage und bildet die Öffnung, welche zum Einführen eines chirurgischen Instrumentes oder eines Fingers vorgesehen ist. Die drei weiteren Seiten des Kreisringsektors sind mit einer Naht unlösbar mit der Folie verbunden. Wie bei den Taschen 25 der Fig. 1 werden die Taschen durch Aufbringen eines flächigen Materialabschnittes auf eine erste flexible Folie hergestellt. Figuren 3a bis 3d illustrieren weitere vorteilhafte Beispiele zur Anordnung der Taschen 25 auf der im Gebrauch von der Wunde abgewandten Seite der Wundauflage 10. Die in Fig. 3 a dargestellte Wundauflage umfasst acht halbkreisförmige Taschen, deren offene Seiten an einem um die Mitte der Wundauflage zentrierten Quadrat ausgerichtet sind und vorwiegend zur Mitte der Wundauflage hin zeigen. Fig. 3b zeigt ein Ausführungsbeispiel, welches eine Anordnung der Taschen 25 auf zwei konzentrischen Kreisumfängen aufweist. Eine derartige Anordnung ist besonders vorteilhaft, da auch nach dem Zuschneiden eines Randbereichs der Wundauflage 10 zur Größenanpassung der Wundauflage weiter innen liegende intakte Taschen vorhanden sind (innerer Kreis von Taschen), während die weiter außen angeordneten Taschen durch das Zuschneiden gegebenenfalls zerstört wurden. Aus der Fig. 3c erkennt man, dass die Wundauflage Taschen unterschiedlicher Größe umfassen kann. Im Falle einer bereits mit Fig. 3b gezeigten Ausführung, welche zwei konzentrische Ringe von Taschen umfasst, könnten beispielsweise die weiter zur Mitte der Wundauflage hin angeordneten Taschen kleiner ausgebildet sein, wie in Fig. 3c schematisch dargestellt. In Weiterbildung dieses Gedankens wird vorgeschlagen die Taschen in einer Vielzahl konzentrischer Ringe um die Mitte der Wundauflage 10 hin anzuordnen, um die Zuschneidbarkeit weiter zu verbessern. Ein solches Beispiel mit vier Ringen konzentrisch auf der Wundauflage angeordneter Taschen ist in Fig. 3d beispielhaft gezeigt. Solchenfalls müssen die Taschen insgesamt kleiner gestaltet werden, beispielsweise mit einer Breite b von 3 cm und einer Tiefe a von 1,5 cm.

In Fig. 4 ist ein Ausschnitt der in Fig. 1 dargestellten Ausführungsform der erfindungsgemäßen Wundauflage 10 im Querschnitt entlang der Linie A-A von Fig. 1 gezeigt. Aus Fig. 4 erkennt man eine erste flexible Folie 1 mit Öffnungen 6. Auf die zweite Seite der Folie 1 wurde ein flächiger Materialabschnitt zur Ausbildung einer Tasche 25 aufgebracht. Der Materialabschnitt ist mit der bogenförmigen Naht 26 (siehe Fig. 1) auf der ersten Folie 1 unlösbar befestigt. Öffnung 27 zeigt zur Mitte der Wundauflage. Gemäß einer vorteilhaften Weiterbildung der Erfindung weist die erste Folie 1 weitestgehend konische oder zylindrische Öffnungen 6 mit einer dreidimensionalen Form auf (in Fig. 4 nicht dargestellt), so dass die Folie infolgedessen eine raue und eine glatte Seite aufweist. Dabei sollte die glatte Seite vorzugsweise die erste Seite der Folie 1 bilden, d.h. als Wundkontaktschicht vorgesehen werden.

Fig. 5 zeigt in stark schematischer Darstellung eine erfindungsgemäße Wundauflage 10, welche neben einer ersten flexiblen Folie 1 eine zweite flexible Folie 4 umfasst.

Analog zu der in Fig. 4 dargestellten Ausführungsform wurde ein flächiger Materialabschnitt zur Ausbildung einer Tasche 25 mittels Naht 26 (siehe Fig. 1) aufgebracht, wobei der Materialabschnitt jedoch auf der zweiten Seite der zweiten Folie 4 vorhanden ist. Erste und zweite Folie (1; 4) sind durch Haftpunkte 7 miteinander verbunden. Haftpunkte 7 können beispielsweise durch punktförmiges Verkleben oder punktförmiges Verschweißen von erster und zweiter Folie erzeugt werden. Bei einer derartigen zweilagigen Ausführung der Wundauflage wird durch die erste Folie 1 und die zweite Folie 4 ein Labyrinth-artiger innerer Hohlraum gebildet, welcher eine Verteilung des Unterdrucks im Wundraum begünstigen kann. Dieser Effekt kann noch verstärkt werden, wenn an den Haftpunkten 7 Material in einer Stärke von beispielsweise 0,1 mm bis 3 mm aufgetragen wird, so dass Abstandhalter zwischen erster und zweiter Folie vorhanden sind. Gemäß einer besonders vorteilhaften Weiterbildung der Erfindung weist sowohl die erste Folie 1 als auch die zweite Folie 4 weitestgehend konische oder zylindrische Öffnungen 6 mit einer dreidimensionalen Form auf (in Fig. 5 nicht dargestellt), so dass, wie vorstehend im Zusammenhang mit Fig. 4 beschrieben, bei jeder der beiden Folien eine glatte Seite und eine der glatten Seite gegenüberliegende aufgeraute Seite vorhanden ist. Es wird nun vorgeschlagen, dass die rauen Seiten von erster Folie 1 und zweiter Folie 4 zueinander in Kontakt gebracht werden. Es entsteht eine durch erste Folie 1 und zweite Folie 4 gebildeter Hohlraum, wobei die dreidimensional geformten Öffnungen eine Beabstandung bewirken. Ein derartiger durch dreidimensionale Strukturen gebildeter Hohlraum bleibt auch während der Unterdruckbehandlung weitgehend erhalten und begünstigt sowohl die Verteilung des Unterdrucks im Wundraum, als auch die Ableitung von Wundexsudat zur Unterdruckquelle. Mit Fig. 6 soll beispielhaft eine alternative Ausgestaltung der Tasche 25 veranschaulicht werden. Man erkennt eine erfindungsgemäße Wundauflage 10 umfassend eine erste flexible Folie 1 und eine zweite flexible Folie 4, wobei die Folienlagen durch Haftpunkte 7 unlösbar miteinander verbunden vorliegen. Zwischen erster Folie 1 und zweiter Folie 4 wird ein Labyrinth-artiger Zwischenraum gebildet. Gemäß der in Fig. 6 gezeigten Ausführung der Erfindung wird eine Tasche 25 durch Rückfaltung der zweiten Folie 4 ausgebildet. Zur Herstellung einer durch Rückfaltung geformten Tasche 25 werden in den Rand der zweiten flexiblen Folie mindestens zwei radial geführte Einschnitte mit einer Tiefe von ca. 3 cm eingebracht. Vorzugsweise wird eine Vielzahl derartige Einschnitte in einem gleichmäßigen Abstand um den vollständigen Rand der zweiten Folie eingebracht. Der Abstand der Einschnitte am Folienrand kann beispielsweise ca. 5 cm betragen. Ein von zwei Einschnitten flankierter Abschnitt der zweiten Folie 4 wird dann auf seine zweite Seite (wundabseits) rückgefaltet, wobei der Abschnitt seitlich unter Bildung einer Naht fixiert wird (in Fig. 6 nicht sichtbar). Eine solchermaßen hergestellte Tasche weist eine zur Mitte der Wundauflage hin orientierte Öffnung auf. Gemäß einer einfacheren Ausführung (nicht dargestellt) der erfindungsgemäßen Wundauflage 10 umfasst die Wundauflage lediglich eine einzige flexible Folie 1, wobei die mindestens eine Tasche durch Rückfaltung der ersten flexiblen Folie 1 gebildet wird. Im Falle einer zweilagig ausgebildeten Wundauflage wäre es auch denkbar, dass erste Folie 1 und zweite Folie 4 gleichzeitig rückgefaltet werden, um eine Tasche zu bilden (nicht dargestellt).

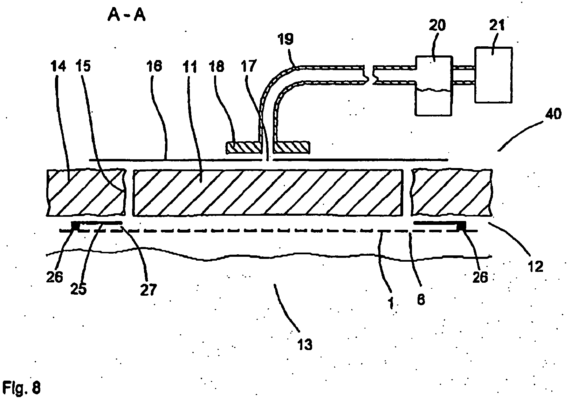

Mit Fig. 7 wird eine weitere vorteilhafte Ausgestaltung einer erfindungsgemäßen Wundauflage 10 vorgestellt. Anstelle eines flächigen Materialabschnitts (siehe Fig. 4; 5) wird auf der im Gebrauch von der Wunde abgewandten Seite der Wundauflage mindestens ein tütenartig ausgebildeter Materialabschnitt, insbesondere einen tütenartig ausgebildeter Folienabschnitt, mittels einer Klebeschicht 28 unlösbar aufgebracht. Bei der in Fig. 8 beispielhaft gezeigten Ausführung liegt eine vollständige Tasche 25 auf einer ersten flexiblen Folie 1 vor. Die Öffnung 27 der Tüte zeigt zur Mitte der Wundauflage 10. Eine tütenartig geformte Tasche könnte ebenso auf die zweite Seite einer zweiten flexiblen Folie (in Fig. 8 nicht dargestellt) oder auf die im Gebrauch von der Wunde abgewandte Seite einer oder mehrerer weiterer Folienlagen aufgebracht werden.

Fig. 8 zeigt eine an eine abdominale Wunde angelegte Vorrichtung 40 zur Unterdrucktherapie von Wunden in stark schematischer Darstellung. Die Vorrichtung umfasst eine wie vorstehend näher beschriebene erfindungsgemäße Wundauflage 10 mit erster Folie 1 und Taschen 25, welche das gleichmäßige Aufbringen und Auslegen der Wundauflage 10 auf den Wundgrund 13 erleichtert. Die erste Seite der ersten Folie 1 wird auf den Wundgrund 13, insbesondere auf freigelegte innere Organe oder auf das Omentum majus, aufgebracht. Erste Folie 1 und die darauf aufgebrachten Taschen 25 werden üblicherweise in den zwischen Wundgrund 13 und Bauchdecke 14 gebildeten Zwischenraum 12 eingeschoben. Das Einschieben der Wundauflage in den zwischen Wundgrund 13 und Bauchdecke 14 gebildeten Zwischenraum 12 wird durch die zur Mitte der Wundauflage hin offenen Taschen 25 erleichtert, da der behandelnde Arzt einen Bauch- oder Darmspatel oder gegebenenfalls einen Finger in die Taschen einführen und sodann den Randbereich der Wundauflage zwischen Bauchdecke 14 und den Wundgrund 13 einbringen kann.

Auf der im Gebrauch von der Wunde abgewandten Seite der Wundauflage liegt eine flüssigkeitsdurchlässige Schicht 11 vor, bei der sich insbesondere um einen porösen Polymerschaumstoff handelt. Vorzugsweise wird ein offenzelliger Polyurethan-Schaumstoff verwendet. Je nach Tiefe der Wunde können mehrere Schichten der flüssigkeitsdurchlässigen Schicht 11 vorhanden sein (nicht dargestellt). Die flüssigkeitsdurchlässige Schicht 11 wurde durch Zuschneiden an die Größe der Wunde angepasst, so dass der Rand der flüssigkeitsdurchlässige Schicht 11 in direktem Kontakt mit dem Wundrand 15 steht. Es ist bekannt, dass der Kontakt des Wundrandes 15 mit einem porösen Polymerschaum 11 das Wachstum des Wundrandgewebes fördert. Die Vorrichtung 40 umfasst weiterhin ein luftundurchlässiges Abdeckmaterial 16 zum luftdichten Verschließen der Wunde, sowie ein auf das luftundurchlässige Abdeckmaterial 16 auf der von der Wunde abgewandten Seite aufgebrachtes Unterdruck-Anschlussstück 18 (Port) zur funktionellen Verbindung des Wundraums mit einer außerhalb des Abdeckmaterials 16 befindlichen Unterdruckquelle 21. Das Unterdruck-Anschlussstück 18 wird im Bereich einer in das luftundurchlässige Abdeckmaterial 16 eingebrachten Öffnung 17 befestigt. Bei Betrieb der Unterdruckquelle 21 (beispielsweise eine Unterdruckpumpe), welche über einen Auffangkanister für Wundexsudat 20 und einer Unterdruckleitung 19 mit dem Port 18 Fluid-leitend verbunden ist, kann Unterdruck im Wundraum hergestellt und Flüssigkeiten aus dem Wundraum abgesaugt werden. Aus dem Wundraum Ober die Unterdruckleitung 19 abgesaugtes Wundexsudat wird im Kanister 20 gesammelt. Zwischen Kanister 20 und Unterdruckquelle 21 ist zweckmäßigerweise ein Filter vorgesehen (nicht dargestellt). Bei Anlegen eines Unterdrucks kann Wundexsudat vom Wundgrund 13 über Öffnungen 6 in der ersten Folie 1 zur flüssigkeitsdurchlässigen Schicht 11 gelangen. Aus der flüssigkeitsdurchlässigen Schicht 11 erfolgt ein Abtransport des Fluids durch eine Öffnung 17 im Abdeckfilm 16 zum Unterdruck-Anschlussstück 18 und durch die Unterdruckleitung 19 weiter zum Kanister 20.

### Anwendung der Wundauflage

Bei der Anwendung zur Unterdrucktherapie großflächiger Wunden im Abdominalbereich wird die erfindungsgemäße Wundauflage 10 zunächst auf den dem Anwender zugänglichen Anteil des Wundgrunds 13 gelegt. Der Rand der Wundauflage 10 wird sodann unter Zuhilfenahme der mindestens einen auf der Wundauflage vorhandenen Tasche ca. 1 bis 15 cm tief in den von Bauchdecke 14 und Wundgrund 13 gebildeten Zwischenraum eingeschoben. Die Wundauflage bildet somit eine für Wundfluid durchlässige Schutzschicht für die freiliegenden inneren Organe. Vorzugsweise wird auf die aus einer oder mehreren Folien gebildete Schutzschicht eine oder mehrere flüssigkeitsdurchlässige Schichten 11, insbesondere Schichten aus einem porösen Polymerschaumstoff, aufgebracht. Dabei ist es für die Wundheilung sehr förderlich, wenn die flüssigkeitsdurchlässige Schicht 11 dergestalt an die Form der Wunde angepasst wird, dass die Wundränder 15 in einem vollständigen Kontakt mit der einen oder mehreren flüssigkeitsdurchlässigen Schicht 11 stehen.

Zum luftdichten Verschließen des Wundbereichs wird ein luftundurchlässiges Abdeckmaterial 16 über die Wunde gelegt. Die Ränder des Abdeckmaterials 16 werden auf die intakte Haut geklebt. Des Weiteren wird ein Unterschlussanschlussstück 18 angebracht, um eine funktionelle Verbindung des Wundraums mit einer außerhalb des Abdeckmaterials 16 befindlichen Unterdruckquelle 21, beispielsweise einer Unterdruckpumpe, herzustellen, so dass ein Unterdruck im Wundraum herstellbar ist und Flüssigkeiten aus dem Wundraum absaugbar sind. Das Unterdruckanschlussstück 18 wird vorzugsweise auf die von der Wunde abgewandte Außenseite des Abdeckmaterials 16 aufgeklebt, wobei vor dem Aufkleben in das ansonsten luftundurchlässige Abdeckmaterial 16 eine geeignete Öffnung 17 geschnitten wird. Die Unterdrucktherapie wird eingeleitet, indem das Unterdruckanschlussstück 18 mit einer Unterdruckquelle 21 verbunden wird und ein vorzugsweise konstanter Unterdruck für eine Dauer von einigen Minuten bis zu mehreren Tagen angelegt wird.

Ein bevorzugter Unterdruck ist der Bereich von mindestens 80 mm Hg bis höchstens 250 mm Hg, vorzugsweise 125 mm Hg.

Entsprechend wird mit der vorliegenden Erfindung ein Verfahren zur Unterdrucktherapie einer Wunde, insbesondere einer Wunde im Abdominalbereich, beschrieben, umfassend die Schritte
a) Auflegen einer erfindungsgemäßen Wundauflage nach einem der Ansprüche 1 bis 17 auf den Wundgrund, insbesondere auf freigelegte innere Organe oder auf das Omentum majus,
b) Abdichtung der Wunde unter Verwendung einer geeigneten luftdichten Abdeckung 16,
c) optional Anbringen eines Unterdruckanschlussmittels 18,
d) Herstellung einer Fluid-Kommunikation mit einer Unterdruckquelle
e) Anlegen von Unterdruck für mindestens 30 Minuten und für höchstens 5 Tage.

## Patentansprüche

1. Wundauflage (10) zur Verwendung bei der Unterdrucktherapie abdominaler Wunden umfassend
i) eine erste flexible Folie (1) mit einer ersten und einer zweiten Seite, wobei die erste Seite zum Aufbringen auf den Wundgrund (13) vorgesehen ist, und wobei es sich bei dem Wundgrund um freigelegte innere Organe oder um das Omentum majus handelt, und wobei die erste Folie (1) weiterhin mindestens eine Öffnung (6) aufweist,
ii) mindestens eine vorwiegend zur Mitte der Wundauflage (10) hin offene Tasche (25), welche auf der im Gebrauch von der Wunde abgewandten Seite der Wundauflage vorhanden ist und welche das gleichmäßige Aufbringen und Auslegen der Wundauflage (10) auf den Wundgrund (13) erleichtert,
**dadurch gekennzeichnet,**
**dass** kein auf die zweite Seite der ersten Folie (1) aufgebrachtes Leitungsmittel aus einem biegsamen elastomeren Material mit einer Dickenerstreckung (H) von höchstens 30 mm, welches einen durchgehenden Hohlraum aufweist, vorhanden ist.

2. Wundauflage (10) nach Anspruch 1, wobei die Tasche (25) einen tütenartig ausgebildeten Materialabschnitt umfasst.

3. Wundauflage (10) nach Anspruch 1, wobei die Tasche (25) durch das Aufbringen eines flächigen Materialabschnittes auf die im Gebrauch von der Wunde abgewandte Seite der Wundauflage (10) gebildet wird.

4. Wundauflage (10) nach einem der vorangehenden Ansprüche, wobei die Tasche (25) auf der zweiten Seite der ersten Folie (1) vorhanden ist.

5. Wundauflage (10) nach einem der Ansprüche 1 bis 3, umfassend eine zweite flexible Folie (4) mit einer ersten und einer zweiten Seite, wobei die erste Seite der zweiten Folie (4) auf die zweite Seite der ersten Folie (1) aufgebracht ist, und wobei die zweite Folie (4) weiterhin mindestens eine Öffnung (6) aufweist.

6. Wundauflage (10) nach Anspruch 5, wobei die Tasche (25) auf der zweiten Seite der zweiten Folie (4) vorhanden ist.

7. Wundauflage (10) nach Anspruch 5, umfassend eine oder mehrere weitere flexible Folien, welche auf der im Gebrauch von der Wunde abgewandten Seite der Wundauflage (10) vorhanden sind.

8. Wundauflage (10) nach Anspruch 7, wobei die Tasche (25) auf der im Gebrauch von der Wunde abgewandten Seite derjenigen Folienlage vorhanden ist, welche im Gebrauch von der Wunde abgewandt vorliegt.

9. Wundauflage (10) nach einem der Ansprüche 1, 5, oder 7, wobei die Tasche durch Rückfaltung eines Folienanteils der Wundauflage gebildet wird.

10. Wundauflage (10) nach einem der vorangehenden Ansprüche, wobei die erste flexible Folie (1) und/oder die zweite flexible Folie (4) und/oder die eine oder die mehreren weiteren flexiblen Folienlage und/oder ein zur Bildung einer Tasche vorgesehener Materialabschnitt eine Vielzahl von über die Fläche verteilten Öffnungen (6) aufweist.

11. Wundauflage (10) nach einem der vorangehenden Ansprüche, wobei die Summe der offenen Fläche der in der ersten flexiblen Folie (1) und/oder der in der zweiten flexiblen Folie (4) und/oder der in der einen oder mehreren weiteren flexiblen Folienlagen und/oder in dem zur Bildung einer Tasche vorgesehenen flächigen Materialabschnitt vorhandenen Öffnungen (6) mindestens 0,1 % und höchstens 25 % der Flächenerstreckung beträgt.

12. Wundauflage (10) nach einem oder mehreren der vorangehenden Ansprüche, wobei die in der ersten flexiblen Folie (1) und/oder die in der zweiten flexiblen Folie (4) und/oder die in der einen oder in den mehreren weiteren flexiblen Folienlagen vorhandenen Öffnungen (6) eine weitestgehend konische oder zylindrische dreidimensionale Form aufweisen und die Folie infolgedessen eine glatte Seite und eine der glatten Seite gegenüberliegende aufgeraute Seite aufweist.

13. Wundauflage (10) nach Anspruch 12, wobei die in der ersten flexiblen Folie (1) vorhandenen Öffnungen (6) eine weitestgehend konische oder zylindrische dreidimensionale Form aufweisen, und wobei die zum Aufbringen auf den Wundgrund (13) vorgesehene erste Seite der ersten flexiblen Folie (1) durch die glatte Seite der ersten Folie (1) gebildet wird und die zweite Seite der ersten Folie (1) durch die aufgeraute Seite gebildet wird.

14. Wundauflage (10) nach einem der vorangehenden Ansprüche, wobei die Wundauflage (10) mindestens 3 Taschen (25) umfasst.

15. Wundauflage (10) nach Anspruch 14, wobei die Taschen (25) auf einem oder mehreren konzentrischen, den Mittelpunkt der Wundauflage umlaufenden Kreisen angeordnet sind.

16. Wundauflage (10) nach einem der vorangehenden Ansprüche, wobei die mindestens eine Tasche (25) im Wesentlichen die Form eines Rechtecks, eines Trapez, eines Halbrunds, eines Dreiecks, eines Kreisrings oder eines Kreisringsektors aufweist.

17. Wundauflage (10) nach einem der vorangehenden Ansprüche, weiterhin umfassend eine oder mehrere flüssigkeitsdurchlässige Schichten (11) zum Aufbringen auf die im Gebrauch von der Wunde abgewandte Seite der Wundauflage.

18. Vorrichtung (40) zur Verwendung bei der Unterdrucktherapie von Wunden umfassend eine Wundauflage nach einem der Ansprüche 1 bis 17, ein luftdichtes Abdeckmaterial (16) zum luftdichten Verschließen der Wunde und der Wundumgebung und ein auf das luftundurchlässige Abdeckmaterial (16) auf der von der Wunde abgewandten Seite aufbringbares Mittel (18) zur funktionellen Verbindung des Wundraums mit einer außerhalb des Abdeckmaterials (16) befindlichen Unterdruckquelle (21), so dass ein Unterdruck im Wundraum herstellbar ist und Flüssigkeiten aus dem Wundraum absaugbar sind.

19. Chirurgisches Instrument zur Applikation einer Wundauflage nach einem oder mehreren der Ansprüche 1 bis 16, wobei das Instrument ein Spatel-artig ausgebildetes, in die Tasche (25) der Wundauflage (10) einführbares Endstück aufweist, und wobei das Instrument weiterhin ein flexibles Kunststoffmaterial und ein Mittel zum Erzeugen eines Röntgenkontrasts umfasst.

20. Gebrauchsfertiges Set zur Unterdruck-Wundbehandlung umfassend eine Vorrichtung nach Anspruch 18, wobei die Komponenten steril verpackt vorliegen.

## Claims

1. Wound dressing (10) for use in negative-pressure therapy of abdominal wounds, comprising
i) a first flexible film (1) with a first and a second side, wherein the first side is provided for application to the wound bed (13), and wherein the wound bed is one or more exposed internal organs or the greater omentum, and wherein the first film (1) furthermore has at least one opening (6),
ii) at least one pocket (25), which is open predominantly towards the centre of the wound dressing (10), is present on the side of the wound dressing facing away from the wound during use and simplifies the uniform application and placement of the wound dressing (10) on the wound bed (13),
**characterized in that**
there is no conduit present which is applied to the second side of the first film (1), is made of a flexible elastomeric material with a thickness (H) of at most 30 mm and has a continuous cavity.

2. Wound dressing (10) according to Claim 1, wherein the pocket (25) comprises a cone-like material portion.

3. Wound dressing (10) according to Claim 1, wherein the pocket (25) is formed by applying a planar material portion to the side of the wound dressing (10) facing away from the wound during use.

4. Wound dressing (10) according to one of the preceding claims, wherein the pocket (25) is present on the second side of the first film (1).

5. Wound dressing (10) according to one of Claims 1 to 3, comprising a second flexible film (4) with a first and a second side, wherein the first side of the second film (4) is applied to the second side of the first film (1), and wherein the second film (4) furthermore has at least one opening (6).

6. Wound dressing (10) according to Claim 5, wherein the pocket (25) is present on the second side of the second film (4).

7. Wound dressing (10) according to Claim 5, comprising one or more further flexible films, which are present on the side of the wound dressing (10) facing away from the wound during use.

8. Wound dressing (10) according to Claim 7, wherein the pocket (25) is present on the side, facing away from the wound during use, of that film ply which faces away from the wound during use.

9. Wound dressing (10) according to one of Claims 1, 5 or 7, wherein the pocket is formed by folding back a film portion of the wound dressing.

10. Wound dressing (10) according to one of the preceding claims, wherein the first flexible film (1) and/or the second flexible film (4) and/or the one or more further flexible film plies and/or a material portion provided for formation of a pocket have/has a multiplicity of openings (6) distributed across the surface.

11. Wound dressing (10) according to one of the preceding claims, wherein the sum of the open surface area of the openings (6) present in the first flexible film (1) and/or in the second flexible film (4) and/or in the one or more further flexible film plies and/or in the planar material portion provided for formation of a pocket is at least 0.1% and at most 25% of the surface extent.

12. Wound dressing (10) according to one or more of the preceding claims, wherein the openings (6) present in the first flexible film (1) and/or in the second flexible film (4) and/or in the one or more further flexible film plies have a substantially conical or cylindrical three-dimensional shape and, as a result of this, the film has a smooth side and a roughened side opposite the smooth side.

13. Wound dressing (10) according to Claim 12, wherein the openings (6) present in the first flexible film (1) have a substantially conical or cylindrical three-dimensional shape, and wherein the first side of the first flexible film (1) provided for application to the wound bed (13) is formed by the smooth side of the first film (1), and the second side of the first film (1) is formed by the roughened side.

14. Wound dressing (10) according to one of the preceding claims, wherein the wound dressing (10) comprises at least 3 pockets (25).

15. Wound dressing (10) according to Claim 14, wherein the pockets (25) are arranged on one or more concentric circles extending around the centre point of the wound dressing.

16. Wound dressing (10) according to one of the preceding claims, wherein the at least one pocket (25) has substantially the shape of a rectangle, a trapezium, a semicircle, a triangle, an annulus or an annular sector.

17. Wound dressing (10) according to one of the preceding claims, furthermore comprising one or more liquid-permeable layers (11) for application to the side of the wound dressing facing away from the wound during use.

18. Device (40) for use in negative-pressure therapy of wounds, comprising a wound dressing according to one of Claims 1 to 17, an airtight cover material (16) for airtight closure of the wound and the wound surroundings, and a means (18) which can be applied to the air-impermeable cover material (16), on the side facing away from the wound, and which permits functional connection of the wound space to a negative-pressure source (21) situated outside the cover material (16), such that a negative pressure can be established in the wound space and liquids can be suctioned out of the wound space.

19. Surgical instrument for applying a wound dressing according to one or more of Claims 1 to 16, wherein the instrument has an endpiece with a spatula-like design that can be inserted into the pocket (25) of the wound dressing (10), and wherein the instrument furthermore comprises a flexible plastic material and a means for generating an X-ray contrast.

20. Ready-to-use kit for negative-pressure wound treatment, comprising a device according to Claim 18, wherein the components are present in a sterile packaged form.

## Revendications

1. Pansement (10) à utiliser dans la thérapie sous dépression de plaies abdominales, comprenant:
i) un premier film flexible (1) présentant une première face et une deuxième face, dans lequel la première face est prévue pour être appliquée sur le fond de plaie (13), et dans lequel le fond de plaie est constitué par des organes internes exposés ou par le grand épiploon et dans lequel le premier film (1) présente en outre au moins une ouverture (6),
ii) au moins une poche (25) ouverte essentiellement vers le milieu du pansement (10), qui est présente sur la face du pansement située en utilisation à l'opposé de la plaie et qui facilite la pose et l'application uniformes du pansement (10) sur le fond de plaie (13),
**caractérisé en ce qu'**il ne se trouve pas de moyen de guidage en une matière élastomère flexible, déposé sur la deuxième face du premier film (1), avec une extension en épaisseur (H) de 30 mm au maximum, qui présente une cavité continue.

2. Pansement (10) selon la revendication 1, dans lequel la poche (25) comprend une pièce de matière réalisée en forme de sachet.

3. Pansement (10) selon la revendication 1, dans lequel la poche (25) est formée par la pose d'une pièce de matière plate sur la face du pansement (10) située en utilisation à l'opposé de la plaie.

4. Pansement (10) selon l'une quelconque des revendications précédentes, dans lequel la poche (25) est présente sur la deuxième face du premier film (1).

5. Pansement (10) selon l'une quelconque des revendications 1 à 3, comprenant un deuxième film flexible (4) avec une première face et une deuxième face, dans lequel la première face du deuxième film (4) est appliquée sur la deuxième face du premier film (1), et dans lequel le deuxième film (4) présente en outre au moins une ouverture (6).

6. Pansement (10) selon la revendication 5, dans lequel la poche (25) est présente sur la deuxième face du deuxième film (4).

7. Pansement (10) selon la revendication 5, comprenant un ou plusieurs autre(s) film(s) flexible(s), qui est/sont présent(s) sur la face du pansement (10) située en utilisation à l'opposé de la plaie.

8. Pansement (10) selon la revendication 7, dans lequel la poche (25) est présente sur la face, située en utilisation à l'opposé de la plaie, de la couche de film située en utilisation à l'opposé de la plaie.

9. Pansement (10) selon l'une quelconque des revendications 1, 5 ou 7, dans lequel la poche est formée en repliant une partie de film du pansement.

10. Pansement (10) selon l'une quelconque des revendications précédentes, dans lequel le premier film flexible (1) et/ou le deuxième film flexible (4) et/ou la ou les autre(s) couche(s) de film flexible et/ou une pièce de matière prévue pour la formation d'une poche présente une multiplicité d'ouvertures (6) réparties sur la surface.

11. Pansement (10) selon l'une quelconque des revendications précédentes, dans lequel la somme de la surface ouverte des ouvertures (6) présentes dans le premier film flexible (1) et/ou dans le deuxième film flexible (4) et/ou dans la ou les plusieurs autre(s) couche(s) de film flexible et/ou dans la pièce de matière plate prévue pour la formation d'une poche vaut au moins 0,1 % et au plus 25 % de l'étendue de la surface.

12. Pansement (10) selon une ou plusieurs des revendications précédentes, dans lequel les ouvertures (6) présentes dans le premier film flexible (1) et/ou dans le deuxième film flexible (4) et/ou dans la ou les plusieurs autre(s) couche(s) de film flexible présentent une forme tridimensionnelle très largement conique ou cylindrique et le film présente par conséquent une face lisse et une face rugueuse opposée à la face lisse.

13. Pansement (10) selon la revendication 12, dans lequel les ouvertures (6) présentes dans le premier film flexible (1) présentent une forme tridimensionnelle très largement conique ou cylindrique, et dans lequel la première face du premier film flexible (1) prévue pour être appliquée sur le fond de plaie (13) est formée par la face lisse du premier film (1) et la deuxième face du premier film (1) est formée par la face rugueuse.

14. Pansement (10) selon l'une quelconque des revendications précédentes, dans lequel le pansement (10) comprend au moins trois poches (25).

15. Pansement (10) selon la revendication 14, dans lequel les poches (25) sont disposées sur un ou sur plusieurs cercles concentriques, entourant le point central du pansement.

16. Pansement (10) selon l'une quelconque des revendications précédentes, dans lequel ladite au moins une poche (25) présente essentiellement la forme d'un rectangle, d'un trapèze, d'un demi-cercle, d'un triangle, d'un anneau circulaire ou d'un secteur d'anneau circulaire.

17. Pansement (10) selon l'une quelconque des revendications précédentes, comprenant en outre une ou plusieurs couche(s) (11) perméable(s) au liquide, à appliquer sur la face du pansement située en utilisation à l'opposé de la plaie.

18. Dispositif (40) à utiliser dans la thérapie sous dépression de plaies, comprenant un pansement selon l'une quelconque des revendications 1 à 17, une matière de recouvrement hermétique (16) pour la fermeture hermétique de la plaie et des alentours de la plaie, et un moyen (18) applicable sur la matière de recouvrement hermétique (16) sur le côté situé à l'opposé de la plaie pour la liaison fonctionnelle de la plaie avec une source de dépression (21) disposée à l'extérieur de la matière de recouvrement (16), de telle sorte qu'une dépression puisse être créée dans la plaie et que des liquides puissent être aspirés hors de la plaie.

19. Instrument chirurgical pour l'application d'un pansement selon une ou plusieurs des revendications 1 à 16, dans lequel l'instrument présente une pièce d'extrémité réalisée en forme de spatule, apte à être introduite dans la poche (25) du pansement (10), et dans lequel l'instrument comprend en outre un matériau plastique flexible et un moyen pour produire un contraste de rayons X.

20. Ensemble prêt à l'emploi pour le traitement de plaies sous dépression, comprenant un dispositif selon la revendication 18, dans lequel les composants se trouvent sous emballage stérile.
